# EUROPEAN PATENT APPLICATION

(11) **EP 2 740 351 A1**
(43) Date of publication of application: **11.06.2014**
(21) Application number: 12196363.1
(22) Date of filing: 10.12.2012
(51) Int. Cl.: A01H 5/08, C07K 14/415, C12N 15/82

(54) **Gene and method for increasing disease resistance in perennial plants**

(71) Applicant: Inova Fruit B.V., 4191 NX Geldermalsen (NL)
(72) Inventor: Schouten, Hendrik Jacob, 6721 HM Bennekom (NL)
(74) Representative: De Hoop, Eric

(57) **Abstract**

This invention relates to method for increasing resistance against pathogen or parasites in a perennial plant, in particular an apple plant. The invention also relates an isolated polynucleotide for use in this method. In the method of the invention a perennial plant with increased resistance against pathogens or parasites is obtained by incorporating into the genome of said perennial plant a gene that confers resistance against pathogens or parasites, wherein said gene is preferably derived from a plant that is capable of natural breeding with said perennial plant.

In particular, this invention relates to a method for increasing resistance to apple scab by introducing Rvi15 (Vr2).

SEQUENCE LISTING

<110> Inova Fruit B.V.

<120> Gene and method for increasing disease resistance in perennial plants

<130> EPP192506

<160> 53

<170> BiSSAP 1.2

<210> 1
<211> 1095
<212> PRT
<213> Malus sp.

<220>
<223> Predicted protein encoded by Vr2-C gene

<400> 1
Met Thr Ala His Glu Ala Ser Ser Ser Ser Ser Ser Lys Ser Lys Leu
1               5                   10                  15
Trp Ser Tyr Asp Val Phe Leu Ser Phe Arg Gly Glu Asp Thr Arg Asn
            20                  25                  30
Gly Phe Thr Ser His Leu His Glu Ala Leu Lys Tyr Arg Gly Tyr His
        35                  40                  45
Val Phe Ile Asp Glu Asp Gly Leu Lys Arg Gly Glu Glu Ile Lys Glu
    50                  55                  60
Lys Leu Phe Arg Ala Ile Glu Glu Ser Arg Ile Ser Ile Ile Val Phe
65                  70                  75                  80
Ser Lys Met Tyr Ala Asp Ser Ser Trp Cys Leu Asp Glu Leu Val Lys
                85                  90                  95
Ile Met Glu Cys Arg Asp Lys Leu Gly Gln His Val Leu Pro Ile Phe
            100                 105                 110
Tyr His Val Asp Pro Ser His Val Arg Lys Gln Asp Gly Asp Leu Ala
        115                 120                 125
Glu Ala Phe Gln Lys His Glu Lys Asp Ile Arg Glu Glu Lys Asp Asp
    130                 135                 140
Glu Lys Arg Glu Ala Lys Gln Lys Arg Ala Glu Gln Trp Lys Gly Ala
145                 150                 155                 160
Leu Thr Lys Ala Ala Asp Leu Ser Gly His His Leu Lys Ser Thr His
                165                 170                 175
Asn Arg Glu Arg Arg Arg Asp Pro Leu Thr Glu Ala Ala Asn Leu Ser
            180                 185                 190
Asp His Gln Thr Thr Asp Asn Gly Ser Arg Lys Gly Lys Thr Phe Pro
        195                 200                 205
Leu Arg Ile Thr Thr Phe Asn Arg Cys Glu Ala Lys Phe Ile Lys Lys
    210                 215                 220
Ile Val Asp Glu Asn Ile Trp Glu Trp Leu Pro Arg Thr Asn Glu Leu
225                 230                 235                 240
His Val Ala Lys His Pro Ile Gly Ile Lys Ser Arg Ile Gln Gly Ile
                245                 250                 255
Ile Ser Asp Leu Ser Ser Gly Gly Ser Asn Asp Val Leu Met Val Gly
            260                 265                 270
Ile Trp Gly Met Gly Gly Leu Gly Lys Thr Thr Val Ala Lys Ala Ile
        275                 280                 285
Tyr Asn Gln Ile His Arg Lys Phe Glu Phe Lys Ser Phe Leu Ala Asp
    290                 295                 300
Val Ser Asp Ala Thr Ser Lys His Gly Leu Val Tyr Leu Gln Glu Lys
305                 310                 315                 320
Leu Ile Ser Asp Ile Leu Lys Gln Lys Ser Gly Ser Ser Asn Phe Asp
                325                 330                 335
Glu Gly Ile Asn Leu Ile Lys Gln His Leu Gln Arg Arg Arg Val Leu
            340                 345                 350

Val Ile Ile Asp Asn Ile Asp Asp Leu Lys Gln Leu Asp Ala Ile Ala
        355                 360                 365
Val Ser His His Cys Phe Gly Pro Gly Ser Arg Ile Ile Ile Thr Thr
    370                 375                 380
Arg Asn Glu His Leu Leu Lys Gln Val Glu Val Asp Lys Thr Tyr Pro
385                 390                 395                 400
Leu Gln Glu Met Lys Lys Lys Glu Ala Leu Glu Leu Phe Ser Trp His
                405                 410                 415
Ala Phe Arg Lys Ser Tyr Pro Asn Glu Glu Tyr Leu Glu Val Ser Glu
            420                 425                 430
Lys Val Val Ser Tyr Cys Gly Gly Leu Pro Leu Ala Leu Glu Val Leu
        435                 440                 445
Gly Ser Phe Leu Phe Lys Arg Pro Ile Ala Glu Trp Lys Ser Gln Leu
    450                 455                 460
Lys Lys Leu Glu Arg Thr Pro Asn Gly Glu Ile Ile Lys Pro Leu Lys
465                 470                 475                 480
Ile Ser Phe Glu Gly Leu Asp Asp Thr Glu Lys Ala Ile Phe Leu Asp
                485                 490                 495
Ile Ser Cys Phe Phe Ile Gly Arg Asp Lys Asp Tyr Val Ala Lys Val
            500                 505                 510
Leu Asp Gly Cys Gly Phe Phe Ala Thr Gly Gly Ile Ser Val Leu Arg
        515                 520                 525
Glu Arg Cys Leu Val Thr Val Glu Gly Asn Lys Leu Thr Met His Asp
    530                 535                 540
Leu Leu Arg Glu Met Ala Arg Val Ile Ile Ser Glu Lys Ser Leu Asp
545                 550                 555                 560
Cys Pro Gly Lys Trp Ser Arg Leu Trp Asn Arg Gln Glu Val Thr Asp
                565                 570                 575
Val Leu Thr Asn Lys Ser Gly Thr Gly Glu Ile Glu Gly Leu Ala Leu
            580                 585                 590
Tyr Phe Pro Lys Glu Thr Lys Ile Ala Ser Phe Ser Thr Lys Ala Phe
        595                 600                 605
Ala Ile Met Lys Lys Leu Ser Leu Leu Ala Leu Leu Phe Arg Lys Glu
    610                 615                 620
Thr Lys Ile Ala Ser Phe Ser Thr Lys Ala Phe Ala Lys Met Lys Lys
625                 630                 635                 640
Leu Arg Leu Leu Gln Leu Ser Asn Val Glu Leu Lys Gly Glu Tyr Lys
                645                 650                 655
His Leu Pro Lys Glu Leu Ile Trp Leu Arg Trp Glu Gly Cys Pro Leu
            660                 665                 670
Lys Ser Leu Pro Asp Asp Phe Phe Asn Gln Pro Arg Leu Val Val Leu
        675                 680                 685
Glu Met Gln Cys Ser Lys Leu Val Gln Val Trp Lys Gly Phe Lys Ser
    690                 695                 700
Leu His Asn Leu Lys Thr Leu Asp Leu Ser Trp Ser Asp Ser Leu Gln
705                 710                 715                 720
Lys Ser Pro Asp Phe Ser Gln Val Pro Asn Leu Glu Glu Leu Ile Leu
                725                 730                 735
Glu Ala Cys Ile Asn Leu Ser Glu Ile His Pro Ser Ile Gly His Leu
            740                 745                 750
Lys Arg Leu Ser Leu Val Asn Leu Lys Trp Cys Asn Lys Leu Ile Ser
        755                 760                 765
Leu Pro Arg Asp Phe Tyr Lys Leu Lys Ser Val Glu Thr Leu Leu Leu
    770                 775                 780
Asn Asn Ser Tyr Gln Phe Arg Glu Val His Glu Asp Ile Gly Glu Met
785                 790                 795                 800
Ile Ser Leu Arg Thr Leu Glu Ala Arg Gly Thr Ala Ile Arg Glu Val
                805                 810                 815
Pro Pro Ser Ile Val Gly Leu Lys Asn Leu Thr Arg Leu Ser Leu Glu
            820                 825                 830
Gly Thr Met Arg Gly Ile Gln Leu Pro His Ser Leu Arg Glu Ile Asp
        835                 840                 845
Leu Thr Tyr Gly Ala Leu Asp Asp Ala Ile Lys Asp Leu Gly Ser Leu
    850                 855                 860
Ile Tyr Leu Gln His Leu His Leu Gln Gly Asn Asn Phe His Ser Leu

865                 870                 875                 880
Pro Asn Leu Ser Gly Leu Leu Lys Leu Glu Thr Leu Trp Leu Asn Gly
                885                 890                 895
Ser Glu Tyr Leu Arg Thr Ile Pro Asp Leu Pro Thr Asn Leu Lys Val
            900                 905                 910
Leu Phe Ala Asp Ala Cys Pro Ala Leu Glu Thr Met Pro Asp Phe Ser
        915                 920                 925
Glu Met Ser Asn Met Arg Thr Leu Asp Val Ser His Ser Ala Lys Leu
    930                 935                 940
Thr Gly Val Pro Gly Leu Asp Lys Ser Leu Asn Ser Met Val Trp Leu
945                 950                 955                 960
Asp Met His Arg Cys Pro Asn Leu Thr Ala Asp Phe Arg Lys Asn Ile
                965                 970                 975
Leu Gln Gly Trp Thr Ser Cys Gly Phe Gly Gly Ile Ala Leu His Gly
            980                 985                 990
Asn Tyr Val Pro Asp Trp Phe Glu Phe Ile Asn Glu Gly Thr Lys Arg
        995                 1000                1005
Thr Lys Leu Arg Ala Tyr Lys Thr Lys Ala Ser Tyr Thr Ile Asp Glu
    1010                1015                1020
Asn Tyr Glu Asn Asp His Leu Trp Gln Gly Gln Phe Ser Asn Asn Glu
1025                1030                1035                1040
Leu Asn Leu Gln Gly Gly Asp Lys Val Asp Ile Val Phe Glu Asn Leu
                1045                1050                1055
Asp Glu Asp Asp Ser Val Lys Met Met Arg Thr Gly Val Asn Leu Val
            1060                1065                1070
Trp Asp Lys Leu Met Lys Glu Asn Met His Asp Leu Gly Asp Thr Arg
        1075                1080                1085
Gly Arg Ile Ser Phe Gly Ile
    1090                1095

<210> 2
<211> 10282
<212> DNA
<213> Malus sp.

<220>
<221> source
<222> 1..10282
<223> /mol_type="unassigned DNA"
      /note="Vr2-C gene with predicted promoter, terminator, exons and
introns"
      /organism="Malus sp."

<220>
<221> CDS
<222>
join(3963..4496,4597..4661,5976..6025,6224..7321,7533..7894,8016..8841,8933..
9018,9145..9411)
<223> /transl_table=1

<220>
<221> promoter
<222> 1..3962

<220>
<221> terminator
<222> 9412..10282

<220>
<221> intron
<222>
join(4497..4596,4662..5975,6026..6223,7322..7532,7895..8015,8842..8932,9019..
9144)

<220>

<221> exon
<222>
join(3963..4496,4597..4661,5976..6025,6224..7321,7533..7894,8016..8841,8933..
9018,9145..9411)

<400> 2
tggttggttc ggtttaagac ttggtgcgaa tcaaactgaa ctatgattat ctctaccaaa 60

tgacttgtta catcaattga catattaatt catagtctaa aattgtaatt tgcctagggt 120

aatgattttc acactcttca tgttgtttat gcctactagt tctcctttat tttgttcaat 180

ataaaagaat ataacaaact caagtgcatg tggacaacaa gagattacag aaattcctct 240

tcgttctcat tcttttgtca tttataactg gtgcattact ccgtttatgt gtacaattca 300

atctataaag ggtatagact gttgatattt gtttgctatg tatataaaca attttatata 360

tggttttcta attcataagg ttgattgtga atattgttaa tagtcgctca ccaatgtgaa 420

ctgctctcca ccaaccagta tataacgaga gaaggaagtt cttgatgcaa tagttgctca 480

caagtcatgt cgattcgagt gaaaccatgc ttgtgtgcca tgcatatctc acctaaaaac 540

gatattattc gcatacaata acaataaata ttagattaat ctcaagtaca tggagatgaa 600

ggaagaattg tccgaactgt tcaaactaaa ccattttaag cagtttgtct tttaatatta 660

tgatctagtg atatttttct tcacttataa atgagaggtc ttaagttcga atctcatgaa 720

agatgagttc gatacaaatt tataccccca cccctaatgt aaatatatct tgtacaaaaa 780

ccaatccaag cagtttggtt tgacttttat aatgaagttt ttagattgaa ctaattcaaa 840

ttttaattag cttggcttgg ttggttcggt ttggcatatt tggaaactgc tgaaaaccaa 900

aactaatttg aacgatgatg ctacattgat tgttgtattg tttaagactt gggccgaatc 960

aaagtgaact acgcttatct ctacaaaatg caattcactt gctaaatcaa ttattattga 1020

taattgacat attaattcac aatcaaaaat tgtaatttcc ttagggtaat gatttttaca 1080

ctcttcatat tgtttatgcc tactagtttt cctttatttt gttcaataaa agaatataac 1140

aaactcaagt gcatgtggac aataagagaa tacataaatt ccttctcgtt cttgttcttt 1200

tgtcatttat aactgataca ttactccatc tatgtgtaca atcgcatcat aaaattccgg 1260

ttcaatttat aaagggtata tactgttgat atttgtttac tatgtatata aacaatttta 1320

tatatggttt tctaattctt atggtagatc gtgaatattg ttaatagtca cttaccaatg 1380

tcaattactc tccaccaacc agtatataac gagagaagaa agttcttgat gcaatagtcg 1440

ctcacaagtc atgtcgattc gagtgaaaac atgcatgtgt gccgtgcata tctcacctac 1500

aaacgatatt gttcgcgtac aataacaata aatattggat taatctcaag tttttttagt 1560

tcaacttaaa gtcataattg ttggacactt tcatatatcc gttaattttt cgtcataacc 1620

ttagttaact aatcacgtag cgtccacata gacaataact gaggccacat gtcaatatgg 1680

accataaata attttaaact tttgacatat ggttttgtac gtatgatcac gttgttctaa 1740

agcatgttga attatgtaaa tgcctttaca gtttggtcaa ttttattgaa atttcaaaca 1800

atatttttgt caaggtgtgc acatcgaaaa cacatggcca caaattccac caattattgg 1860
gtggattaga aggaccaaat taggaatgag tatatttgat ataaaatagg agtggatata 1920
attgaagata aaatgagaaa aagttggtta aaaatgatag acatgtgaac taaaggccta 1980
caaatgttct tgttagaaaa agacggaggg tcaatgtaaa aagggtaaag gaaaacctca 2040
taatacttgg aatttggaaa taaactttaa gaaaagacat ggagtactca aagatgactg 2100
aagacttggt gcaaatccaa acacaatagc gtttcaagag acatatagcc gatcccgctt 2160
agtgggataa gactttatcg cttttgtatt tggtggattg gaaggaatta ttgagttttt 2220
gcaactatat atgaaccagc cgttgtggtt ttacataatt caatgttggg ccttgggcat 2280
tatgttttgc cattaatatc ccaacgacat tgccaactac atatgaacta gcacgcttcg 2340
ctatggtata cttttccagc ggatgagata ggccttgggc attaaaagcg actgcatttt 2400
atggcagtac tcatttttac tttttataca tttttgttaa ttttttgtaa ttattatttt 2460
ttaatttatt tgatctgact atttaagatt aagaaggact tgtcaaaagg aaaaatgatt 2520
gtgtgaagaa attattttaa tgatgaagtg agagggtgag ataggattag atagtcacca 2580
gtattgatct ttcgcgtgtg gacgttgttt gacgcgaagc aagtatgaaa aacaaaacgt 2640
tgccacacga tctgggatgg acgattttat atcaacttaa tggacgtgga tgacttttgc 2700
ttttgttgat tgaaggagct tgacctggat tttgatattt caatatgtac ccaccacaga 2760
agttgccaat gatgtcgatc gtattcatat atgaatcttt tgtgaaactt caatcttagc 2820
catcaacgca ttttttttgg actttctgtt accttatttt cttagcataa tattttataa 2880
tattgatata gaaattatgt aaaaaaatga agtagtcgag agttgatgta aaatcctatt 2940
ttgagagtct ctttgttcct tcaaaagcag ctcctgcatg cttttccaca gtccaattac 3000
gtgtccgtag ggttacatga ccatttttgt ctccctgagc ccaagtttca aaagccgccg 3060
acgactcgaa ttcctcgcaa aatcgtccat actgctttta gtaacgtcct tacctgataa 3120
ataaataaaa aaacacaaac gaaagaccac atttttcttt ctctttatct attttctcag 3180
caaccaaacg gaaaacaaga acaccgttgg tgacctccgg atttgagagg actttcgaag 3240
cgcatgcggg tagggcaagg acctggcgga gatggatttg aggtccatgt tggtgagctt 3300
cgcttccact gaaatcaaca ccgtacgcgt gtcgtcggaa aaggcggact ggatcaggat 3360
catccattaa cttgttcgat gtcgccgaag caccaccctc tgtttcttag gtttcgattg 3420
cggcgtcgga actccccttc atttttttta gcgcctgttt gccgtgttgt ttgatttggg 3480
gtttttctaa ttttgcaacg gaaaacggct aatgtacggt acagtcccaa ataggtcatg 3540
aaacggggag atccggggag gatcactttg ggtgggaaag atccggaaag gatctctttc 3600
catgttcaat aatgtaaatg ccttagcagt aatttcaaac aatatttttg tcaaggcgtg 3660
cacatcgaaa gcacatagcc acaaattcca ccaataattt attgaaatat caaacaatat 3720

tttattgaaa tttcctttct ttcctgcttt ccaccgtcct attacgtgtc ggtagtctgt 3780

cccttcgtgt tcctttcttt cctttttgcc tctctctttc cttgcacctt cctgcccggt 3840

gatttgtcgt ttctcaagtc catccaacat tattattttt cttcagcaac ctaaccacaa 3900

ccattgccat tttttcagtc tctgatctaa accattgcag tgtcataacg gtggataccg 3960

cc atg aca gcc cac gaa gcc tcc tct tcg tcc tcc tct aag tca aaa 4007
Met Thr Ala His Glu Ala Ser Ser Ser Ser Ser Ser Lys Ser Lys
1               5                   10                  15

ctt tgg agt tac gac gtg ttc ttg agc ttc aga ggc gaa gac acg cgg 4055
Leu Trp Ser Tyr Asp Val Phe Leu Ser Phe Arg Gly Glu Asp Thr Arg
                20                  25                  30

aat ggc ttc acc agc cac ctc cac gag gca tta aaa tac aga gga tac 4103
Asn Gly Phe Thr Ser His Leu His Glu Ala Leu Lys Tyr Arg Gly Tyr
            35                  40                  45

cat gtc ttt att gat gag gac ggt cta aaa aga ggg gaa gaa ata aaa 4151
His Val Phe Ile Asp Glu Asp Gly Leu Lys Arg Gly Glu Glu Ile Lys
        50                  55                  60

gag aaa ctg ttt cgt gca atc gaa gag tcg agg atc tct atc att gtc 4199
Glu Lys Leu Phe Arg Ala Ile Glu Glu Ser Arg Ile Ser Ile Ile Val
    65                  70                  75

ttc tca aag atg tat gcg gat tcc agt tgg tgt ctt gac gag ctg gtg 4247
Phe Ser Lys Met Tyr Ala Asp Ser Ser Trp Cys Leu Asp Glu Leu Val
80                  85                  90                  95

aag atc atg gaa tgc aga gac aag ctg ggg caa cat gtt ttg cca ata 4295
Lys Ile Met Glu Cys Arg Asp Lys Leu Gly Gln His Val Leu Pro Ile
                100                 105                 110

ttc tat cac gtt gat cct tcc cat gtc agg aag cag gac gga gat tta 4343
Phe Tyr His Val Asp Pro Ser His Val Arg Lys Gln Asp Gly Asp Leu
            115                 120                 125

gcc gaa gca ttt cag aaa cac gaa aag gac atc cgt gaa gaa aaa gat 4391
Ala Glu Ala Phe Gln Lys His Glu Lys Asp Ile Arg Glu Glu Lys Asp
        130                 135                 140

gac gag aaa cgt gaa gct aaa caa aaa agg gca gag cag tgg aaa ggg 4439
Asp Glu Lys Arg Glu Ala Lys Gln Lys Arg Ala Glu Gln Trp Lys Gly
    145                 150                 155

gct ctc aca aaa gct gca gat ttg tct ggc cac cat ctt aaa tcc act 4487
Ala Leu Thr Lys Ala Ala Asp Leu Ser Gly His His Leu Lys Ser Thr
160                 165                 170                 175

cat aat agg taatcattca actgctgttc aagatttaag ttgtattact 4536
His Asn Arg

ctaattaaca tgcttaatta atacacctta atctctcacg ttgatccttc gcatgtcagg 4596

gag agg cgg aga gat cct ctt acc gaa gct gca aat ttg tct gac cac 4644
Glu Arg Arg Arg Asp Pro Leu Thr Glu Ala Ala Asn Leu Ser Asp His
    180                 185                 190

cag aca act gac aac gg gtaatctttc acttgcgttt caagatttga 4691
Gln Thr Thr Asp Asn Gly
195                 200

aacattttag tgaatgactt aatctctcaa ttaattaaca tgctcaatta acattttaca 4751

taatccaaac attgattcac aggcttaatg aatacacttt aatctctcat ataaaaccta 4811

aaccgttatt ggagtttaat tcatacacaa cttttattta acaatgaatg actttctttc 4871

ttttattcta gtcacatact atctagttgc tagttagcat atatatatag gaaaaattag 4931

attttcatct cttattttta tattgcatcg attgaaactt tattattttt ttatttttga 4991

tcaaggtcct tcgaccttgt ccacatcatc attttagaat ttgaaaaatt agacgtcaac 5051

actttgctaa ttatttatag tttttagtta aggtattagc cctttttttg ttgaaattaa 5111

tattaattgc tttgtccaga ttttaagttg ataattgaaa tatcatctct tcccacattt 5171

aagtcaaata aacagtcatt tatactcctc cattcttttt cattcctcac gcacatgacc 5231

tctaaactca atccatcatg cttgttcatt gtcgagacgc caaccattat atttatgtag 5291

tcaacttttt ttcttctttt tgttttttat tttcctttta ttattaaata ttaatcttct 5351

tcttttttaa agaatattga atattaatac tgattgttaa atatatggtt ggctatactt 5411

ttgtatttca tgggtacaat tttattttac attttatttc attctaatat atctattggt 5471

acaatatatt ttgtgagtac aatttcgtta tacatattga tatgggtact attatttttt 5531

ttggagaaaa ttaggtaaaa tggttgtggt tgcagtattt tttatgtacc atacttatgc 5591

ttactaaaca ttgtgaaaca ttctttgaca aataaaaaaa ataaataaac aatgtggaac 5651

attcttttcc ttgagttgtt tacatgtacc catgagtttt catgcttcat ggacgtttca 5711

aatatcttct tttattttaa gcgtacctga ttagcagaaa acgaataagc cttcgaattt 5771

ggtggatgat agtctcattc ttcgatgcgt caattcctca gcttctggca agagcgtgtt 5831

aataacatgt tgtaagccta tttgattgag tgatcgaggg ttagagagag agagagaaag 5891

gaggtgcggc aatattgagg gagaagagag attgatttaa ttgtgaagtt tgtttgattc 5951

acccaattgt gtctttattt atag t agt aga aag ggt aaa aca ttt ccc ctt 6003
Ser Arg Lys Gly Lys Thr Phe Pro Leu
                205

agg att aca aca ttt aat agg t aatctaatcc taataggaat ataagataca 6055
Arg Ile Thr Thr Phe Asn Arg
210                 215

ttcccagatt ctctaggatt tacacaatca cattcctatt ctaaatatga ctacaacata 6115

tatatatata tatatatatg aagaaaagaa agaaaattgt ttatgtattg atatctccta 6175

cagagcctca ttgagtcaca ctaatgcttc tgaattcaat tttcaggt gc gaa gca 6231
Cys Glu Ala

aag ttc att aaa aaa att gtt gac gag aat att tgg gaa tgg ctc ccc 6279
Lys Phe Ile Lys Lys Ile Val Asp Glu Asn Ile Trp Glu Trp Leu Pro
220                 225                 230                 235

aga aca aac gaa tta cat gtg gcc aag cac cca att gga atc aaa tct 6327
Arg Thr Asn Glu Leu His Val Ala Lys His Pro Ile Gly Ile Lys Ser

240                 245                 250

cgc att caa ggt att atc agt gat ctt tca agt ggt gga tca aat gat      6375
Arg Ile Gln Gly Ile Ile Ser Asp Leu Ser Ser Gly Gly Ser Asn Asp
            255                 260                 265

gtt ctc atg gtt gga att tgg ggg atg ggt gga ttg ggt aaa aca aca      6423
Val Leu Met Val Gly Ile Trp Gly Met Gly Gly Leu Gly Lys Thr Thr
        270                 275                 280

gtt gcc aaa gcc att tat aac caa att cat cgt aag ttt gaa ttc aaa      6471
Val Ala Lys Ala Ile Tyr Asn Gln Ile His Arg Lys Phe Glu Phe Lys
    285                 290                 295

agt ttc ctt gcc gac gtt agc gac gct aca agt aaa cat ggt cta gtt      6519
Ser Phe Leu Ala Asp Val Ser Asp Ala Thr Ser Lys His Gly Leu Val
300                 305                 310                 315

tat ttg caa gaa aaa ctt att tct gac atc ttg aaa cag aag tct gga      6567
Tyr Leu Gln Glu Lys Leu Ile Ser Asp Ile Leu Lys Gln Lys Ser Gly
                320                 325                 330

tca agc aat ttt gat gaa ggt atc aat ttg ata aaa caa cat ctc caa      6615
Ser Ser Asn Phe Asp Glu Gly Ile Asn Leu Ile Lys Gln His Leu Gln
            335                 340                 345

cgt aga agg gta ctt gtc atc atc gac aat ata gat gac ttg aaa caa      6663
Arg Arg Arg Val Leu Val Ile Ile Asp Asn Ile Asp Asp Leu Lys Gln
        350                 355                 360

cta gat gca ata gct gta agt cac cat tgt ttt ggt cca gga agt aga      6711
Leu Asp Ala Ile Ala Val Ser His His Cys Phe Gly Pro Gly Ser Arg
    365                 370                 375

att att ata aca aca cga aat gaa cat ttg cta aag caa gtg gaa gtg      6759
Ile Ile Ile Thr Thr Arg Asn Glu His Leu Leu Lys Gln Val Glu Val
380                 385                 390                 395

gac aag aca tat ccg ctt cag gaa atg aaa aag aaa gaa gcc ctg gag      6807
Asp Lys Thr Tyr Pro Leu Gln Glu Met Lys Lys Lys Glu Ala Leu Glu
                400                 405                 410

ctc ttt agt tgg cat gcc ttt cga aaa agt tac cct aat gaa gaa tat      6855
Leu Phe Ser Trp His Ala Phe Arg Lys Ser Tyr Pro Asn Glu Glu Tyr
            415                 420                 425

ctt gaa gtc tca gaa aag gtt gtt tct tac tgt gga ggt ttg cca cta      6903
Leu Glu Val Ser Glu Lys Val Val Ser Tyr Cys Gly Gly Leu Pro Leu
        430                 435                 440

gca ctt gaa gtt tta ggc tct ttt ttg ttt aaa aga cct att gca gag      6951
Ala Leu Glu Val Leu Gly Ser Phe Leu Phe Lys Arg Pro Ile Ala Glu
    445                 450                 455

tgg aaa agt caa ttg aag aaa ttg gaa aga act cct aat gga gaa ata      6999
Trp Lys Ser Gln Leu Lys Lys Leu Glu Arg Thr Pro Asn Gly Glu Ile
460                 465                 470                 475

ata aaa cca cta aaa ata agc ttt gaa ggg cta gat gat aca gag aag      7047
Ile Lys Pro Leu Lys Ile Ser Phe Glu Gly Leu Asp Asp Thr Glu Lys
                480                 485                 490

gct ata ttc ctt gac ata tct tgt ttc ttt att gga agg gac aag gac      7095
Ala Ile Phe Leu Asp Ile Ser Cys Phe Phe Ile Gly Arg Asp Lys Asp
            495                 500                 505

tac gtt gca aaa gta tta gat gga tgt gga ttt ttt gca aca gga gga     7143
Tyr Val Ala Lys Val Leu Asp Gly Cys Gly Phe Phe Ala Thr Gly Gly
        510                 515                 520

atc agt gtc ctc cgc gaa cga tgt ctt gta act gtt gaa ggc aac aag     7191
Ile Ser Val Leu Arg Glu Arg Cys Leu Val Thr Val Glu Gly Asn Lys
    525                 530                 535

ttg act atg cat gat ttg ctt cga gaa atg gcc aga gta atc att tct     7239
Leu Thr Met His Asp Leu Leu Arg Glu Met Ala Arg Val Ile Ile Ser
540                 545                 550                 555

gaa aaa tct ctt gat tgc cct gga aaa tgg agt agg ttg tgg aat cgt     7287
Glu Lys Ser Leu Asp Cys Pro Gly Lys Trp Ser Arg Leu Trp Asn Arg
                560                 565                 570

caa gag gtc act gat gta ttg aca aat aaa tct g tgagtttatt            7331
Gln Glu Val Thr Asp Val Leu Thr Asn Lys Ser
            575                 580

cccaataaat tttggattta tgcatcatac aagaaaagca tagatgtaac cctcgcaagt   7391

caattataca ttaaacaaga ttcatgtgta aatatattca ccgtgcgtga ttttgcgtaa   7451

tgaataataa taatacatgt aaacatatgc aaatataccg tttcttacca ctataacatt   7511

tttttttttt ttgttaacag g ga  act gga gaa att gaa gga ctt gct cta     7561
                        Gly Thr Gly Glu Ile Glu Gly Leu Ala Leu
                                585                 590

tat ttc cct aaa gaa act aag att gct agt ttc agt aca aaa gca ttt     7609
Tyr Phe Pro Lys Glu Thr Lys Ile Ala Ser Phe Ser Thr Lys Ala Phe
        595                 600                 605

gcc ata atg aag aaa ctg agc ctg ctt gct cta ctt ttc cgt aaa gaa     7657
Ala Ile Met Lys Lys Leu Ser Leu Leu Ala Leu Leu Phe Arg Lys Glu
    610                 615                 620

act aag att gct agt ttc agt aca aaa gca ttt gcc aaa atg aag aaa     7705
Thr Lys Ile Ala Ser Phe Ser Thr Lys Ala Phe Ala Lys Met Lys Lys
625                 630                 635                 640

ctg aga ctg ctt cag ctc agc aac gta gag ctc aaa gga gaa tac aaa     7753
Leu Arg Leu Leu Gln Leu Ser Asn Val Glu Leu Lys Gly Glu Tyr Lys
                645                 650                 655

cat ctt ccc aaa gag tta ata tgg ttg cgt tgg gaa gga tgc cct tta     7801
His Leu Pro Lys Glu Leu Ile Trp Leu Arg Trp Glu Gly Cys Pro Leu
            660                 665                 670

aag tcc tta cca gat gac ttt ttt aat caa cca aga cta gtt gtt tta     7849
Lys Ser Leu Pro Asp Asp Phe Phe Asn Gln Pro Arg Leu Val Val Leu
        675                 680                 685

gag atg cag tgt agc aaa ctg gta caa gtt tgg aag ggt ttc aag         7894
Glu Met Gln Cys Ser Lys Leu Val Gln Val Trp Lys Gly Phe Lys
    690                 695                 700

gtacaatgaa ttacacctga tgatgttctg ttcctatttg ttttttggat ttcttaactt    7954

ttttttgtaa catcttcatt ttggatatta atttcaattt tcttttgatt ttctgcaaca    8014

g tcg cta cat aac ttg aaa acc ctt gat ctc agc tgg tct gat tcc tta 8063
Ser Leu His Asn Leu Lys Thr Leu Asp Leu Ser Trp Ser Asp Ser Leu
    705                 710                 715

cag aaa tca ccg gac ttt tca caa gtc cca aat ctt gaa gag ttg ata 8111
Gln Lys Ser Pro Asp Phe Ser Gln Val Pro Asn Leu Glu Glu Leu Ile
720                 725                 730                 735

ttg gaa gcc tgt att aat ctg tcc gag att cac ccc tcc att ggt cat 8159
Leu Glu Ala Cys Ile Asn Leu Ser Glu Ile His Pro Ser Ile Gly His
                740                 745                 750

ctt aaa aga ctt tct ttg gtg aac ctt aag tgg tgt aat aag ctt att 8207
Leu Lys Arg Leu Ser Leu Val Asn Leu Lys Trp Cys Asn Lys Leu Ile
            755                 760                 765

tct ctt cca agg gat ttc tat aag ttg aaa tct gtt gag act ctt ctt 8255
Ser Leu Pro Arg Asp Phe Tyr Lys Leu Lys Ser Val Glu Thr Leu Leu
        770                 775                 780

ctt aat aat tct tat caa ttc aga gaa gtg cat gag gat ata ggg gag 8303
Leu Asn Asn Ser Tyr Gln Phe Arg Glu Val His Glu Asp Ile Gly Glu
    785                 790                 795

atg ata tca ctg aga aca ctt gaa gca agg ggt aca gcc ata aga gaa 8351
Met Ile Ser Leu Arg Thr Leu Glu Ala Arg Gly Thr Ala Ile Arg Glu
800                 805                 810                 815

gta cca cct tcc ata gta gga ttg aag aat ctc act cgt tta tct ctg 8399
Val Pro Pro Ser Ile Val Gly Leu Lys Asn Leu Thr Arg Leu Ser Leu
                820                 825                 830

gag ggt act atg agg ggt att caa ttg ccc cat tcg tta aga gaa atc 8447
Glu Gly Thr Met Arg Gly Ile Gln Leu Pro His Ser Leu Arg Glu Ile
            835                 840                 845

gat ctc aca tac ggc gca tta gat gat gca att aag gat ctt ggc agt 8495
Asp Leu Thr Tyr Gly Ala Leu Asp Asp Ala Ile Lys Asp Leu Gly Ser
        850                 855                 860

cta att tat tta caa cat ttg cat ctt caa ggt aat aac ttt cat agc 8543
Leu Ile Tyr Leu Gln His Leu His Leu Gln Gly Asn Asn Phe His Ser
    865                 870                 875

tta cca aac ctc agt ggt ctt tta aag ctc gaa aca ttg tgg tta aat 8591
Leu Pro Asn Leu Ser Gly Leu Leu Lys Leu Glu Thr Leu Trp Leu Asn
880                 885                 890                 895

gga agc gaa tat ctt cgt aca atc cct gat tta cca aca aat ttg aaa 8639
Gly Ser Glu Tyr Leu Arg Thr Ile Pro Asp Leu Pro Thr Asn Leu Lys
                900                 905                 910

gtt ctg ttt gcc gat gct tgc cct gca ttg gaa aca atg ccg gac ttt 8687
Val Leu Phe Ala Asp Ala Cys Pro Ala Leu Glu Thr Met Pro Asp Phe
            915                 920                 925

tcg gaa atg tca aat atg aga aca ctg gat gta agt cat tca gcc aaa 8735
Ser Glu Met Ser Asn Met Arg Thr Leu Asp Val Ser His Ser Ala Lys
        930                 935                 940

ctc act ggg gtt cca ggc ttg gat aag tca tta aac tcc atg gtg tgg 8783
Leu Thr Gly Val Pro Gly Leu Asp Lys Ser Leu Asn Ser Met Val Trp
    945                 950                 955

ctt gat atg cat cgc tgc ccc aat ctc aca gct gat ttt agg aag aac 8831

Leu Asp Met His Arg Cys Pro Asn Leu Thr Ala Asp Phe Arg Lys Asn
960                 965                 970                 975

atc cta cag g tatgtctctc tccctatcat tgccttccta tcgtgcgcat        8881
Ile Leu Gln

acatgtgtat atatatatat atatatgtga cacttgtatt gcatatatag g ga tgg   8937
                                                        Gly Trp
                                                            980

act tct tgc gga ttt ggt ggc att gct ctc cat ggg aat tat gtt cct   8985
Thr Ser Cys Gly Phe Gly Gly Ile Ala Leu His Gly Asn Tyr Val Pro
            985                 990                 995

gat tgg ttt gag ttt atc aac gag ggc act aaa gtcagttttg atattccccc   9038
Asp Trp Phe Glu Phe Ile Asn Glu Gly Thr Lys
        1000                1005

gaccgatgat cgtaattttg acgggctaac tctattctgc ttgttccgcg tatgcaaaaa   9098

aaaaagatta cctcatcttg acattaatgt tataattaat accaag cgt acg aag   9153
                                                   Arg Thr Lys
                                                           1010

ttg cgg gcc tac aaa acc aaa gcc tca tat acg atc gat gag aat tat   9201
Leu Arg Ala Tyr Lys Thr Lys Ala Ser Tyr Thr Ile Asp Glu Asn Tyr
            1015                1020                1025

gaa aac gat cat ctt tgg cag gga caa ttc tca aac aat gag ctc aat   9249
Glu Asn Asp His Leu Trp Gln Gly Gln Phe Ser Asn Asn Glu Leu Asn
        1030                1035                1040

ttg caa ggt ggg gat aaa gtt gat ata gtt ttt gaa aat cta gat gaa   9297
Leu Gln Gly Gly Asp Lys Val Asp Ile Val Phe Glu Asn Leu Aso Glu
    1045                1050                1055

gat gat tct gtt aaa atg atg aga aca ggg gtt aat cta gta tgg gac   9345
Asp Asp Ser Val Lys Met Met Arg Thr Gly Val Asn Leu Val Trp Asp
    1060                1065                1070

aaa ctt atg aag gaa aat atg cat gat ttg ggc gac act aga ggt aga   9393
Lys Leu Met Lys Glu Asn Met His Asp Leu Gly Asp Thr Arg Gly Arg
1075                1080                1085                1090

atc agt ttt ggc atc tga taaactcgtt tgtgcagaat gaaggtgaaa        9441
Ile Ser Phe Gly Ile
            1095

gcatcacaat caattcgtca aacacctcac catttttcgc tttcctagtt cgataatgga   9501

ggcgaatcca tatttcatgc ttttattttt tcagattcct atgatttttt caattagttg   9561

ttttggattt tgaatttatt gcatcaagta acatttcaac attacagcaa tcaatataat   9621

cttccgccac atattcaagg aatttacttt taaggataga aagaaaatca atgatagcct   9681

caagatgcca gtcaatggtt tctactacct tttcattaat cttgacttgc cttgacatgt   9741

tagcagaaga ttgactctac atttactaaa agatttatta ccttttctaa tacctttgca   9801

gtcaatgaac tcgtttgcgc aataaaaata tagtcttatg ttggattctc caaaacttta   9861

gaagcaagtt gtttgaaaga ttgtagagct gttcctttta agataaaaaa aaatctcatt   9921

tgcaagactc agaaatgatg gattgctcaa tagaatatct cggaaatttt ctccacttcc 9981

cttccacttt gactgatgag ttcataagct tccttctccg attttagttt ctactagtga 10041

tgctgcaggt tttaaaacca tataaagcat gtcaaaagtt ccaagtatat atacgatatg 10101

caatatttac atacatgtga ttcatgtctt cttaaccatt aacagaacaa actgaaaaaa 10161

agaaaagcaa aaatatgaat tgtacatgaa attgtgttca atcctaaaac tacacttcag 10221

aaggataaat atacatgcgc tctgcaattt catttctcag ttctaccatt gccacttatt 10281

c 10282

<210> 3
<211> 3288
<212> DNA
<213> Malus sp.

<220>
<221> source
<222> 1..3288
<223> /mol_type="unassigned DNA"
/note="Predicted coding sequence of Vr2-C"
/organism="Malus sp."

<220>
<221> CDS
<222> 1..3288
<223> /transl_table=1

<400> 3
atg aca gcc cac gaa gcc tcc tct tcg tcc tcc tct aag tca aaa ctt 48
Met Thr Ala His Glu Ala Ser Ser Ser Ser Ser Ser Lys Ser Lys Leu
1               5                   10                  15

tgg agt tac gac gtg ttc ttg agc ttc aga ggc gaa gac acg cgg aat 96
Trp Ser Tyr Asp Val Phe Leu Ser Phe Arg Gly Glu Asp Thr Arg Asn
            20                  25                  30

ggc ttc acc agc cac ctc cac gag gca tta aaa tac aga gga tac cat 144
Gly Phe Thr Ser His Leu His Glu Ala Leu Lys Tyr Arg Gly Tyr His
        35                  40                  45

gtc ttt att gat gag gac ggt cta aaa aga ggg gaa gaa ata aaa gag 192
Val Phe Ile Asp Glu Asp Gly Leu Lys Arg Gly Glu Glu Ile Lys Glu
    50                  55                  60

aaa ctg ttt cgt gca atc gaa gag tcg agg atc tct atc att gtc ttc 240
Lys Leu Phe Arg Ala Ile Glu Glu Ser Arg Ile Ser Ile Ile Val Phe
65                  70                  75                  80

tca aag atg tat gcg gat tcc agt tgg tgt ctt gac gag ctg gtg aag 288
Ser Lys Met Tyr Ala Asp Ser Ser Trp Cys Leu Asp Glu Leu Val Lys
                85                  90                  95

atc atg gaa tgc aga gac aag ctg ggg caa cat gtt ttg cca ata ttc 336
Ile Met Glu Cys Arg Asp Lys Leu Gly Gln His Val Leu Pro Ile Phe
            100                 105                 110

tat cac gtt gat cct tcc cat gtc agg aag cag gac gga gat tta gcc 384
Tyr His Val Asp Pro Ser His Val Arg Lys Gln Asp Gly Asp Leu Ala
        115                 120                 125

gaa gca ttt cag aaa cac gaa aag gac atc cgt gaa gaa aaa gat gac 432

Glu Ala Phe Gln Lys His Glu Lys Asp Ile Arg Glu Glu Lys Asp Asp
    130                 135                 140

gag aaa cgt gaa gct aaa caa aaa agg gca gag cag tgg aaa ggg gct      480
Glu Lys Arg Glu Ala Lys Gln Lys Arg Ala Glu Gln Trp Lys Gly Ala
145                 150                 155                 160

ctc aca aaa gct gca gat ttg tct ggc cac cat ctt aaa tcc act cat      528
Leu Thr Lys Ala Ala Asp Leu Ser Gly His His Leu Lys Ser Thr His
                165                 170                 175

aat agg gag agg cgg aga gat cct ctt acc gaa gct gca aat ttg tct      576
Asn Arg Glu Arg Arg Arg Asp Pro Leu Thr Glu Ala Ala Asn Leu Ser
            180                 185                 190

gac cac cag aca act gac aac ggt agt aga aag ggt aaa aca ttt ccc      624
Asp His Gln Thr Thr Asp Asn Gly Ser Arg Lys Gly Lys Thr Phe Pro
        195                 200                 205

ctt agg att aca aca ttt aat agg tgc gaa gca aag ttc att aaa aaa      672
Leu Arg Ile Thr Thr Phe Asn Arg Cys Glu Ala Lys Phe Ile Lys Lys
    210                 215                 220

att gtt gac gag aat att tgg gaa tgg ctc ccc aga aca aac gaa tta      720
Ile Val Asp Glu Asn Ile Trp Glu Trp Leu Pro Arg Thr Asn Glu Leu
225                 230                 235                 240

cat gtg gcc aag cac cca att gga atc aaa tct cgc att caa ggt att      768
His Val Ala Lys His Pro Ile Gly Ile Lys Ser Arg Ile Gln Gly Ile
                245                 250                 255

atc agt gat ctt tca agt ggt gga tca aat gat gtt ctc atg gtt gga      816
Ile Ser Asp Leu Ser Ser Gly Gly Ser Asn Asp Val Leu Met Val Gly
            260                 265                 270

att tgg ggg atg ggt gga ttg ggt aaa aca aca gtt gcc aaa gcc att      864
Ile Trp Gly Met Gly Gly Leu Gly Lys Thr Thr Val Ala Lys Ala Ile
        275                 280                 285

tat aac caa att cat cgt aag ttt gaa ttc aaa agt ttc ctt gcc gac      912
Tyr Asn Gln Ile His Arg Lys Phe Glu Phe Lys Ser Phe Leu Ala Asp
    290                 295                 300

gtt agc gac gct aca agt aaa cat ggt cta gtt tat ttg caa gaa aaa      960
Val Ser Asp Ala Thr Ser Lys His Gly Leu Val Tyr Leu Gln Glu Lys
305                 310                 315                 320

ctt att tct gac atc ttg aaa cag aag tct gga tca agc aat ttt gat     1008
Leu Ile Ser Asp Ile Leu Lys Gln Lys Ser Gly Ser Ser Asn Phe Asp
                325                 330                 335

gaa ggt atc aat ttg ata aaa caa cat ctc caa cgt aga agg gta ctt     1056
Glu Gly Ile Asn Leu Ile Lys Gln His Leu Gln Arg Arg Arg Val Leu
            340                 345                 350

gtc atc atc gac aat ata gat gac ttg aaa caa cta gat gca ata gct     1104
Val Ile Ile Asp Asn Ile Asp Asp Leu Lys Gln Leu Asp Ala Ile Ala
        355                 360                 365

gta agt cac cat tgt ttt ggt cca gga agt aga att att ata aca aca     1152
Val Ser His His Cys Phe Gly Pro Gly Ser Arg Ile Ile Ile Thr Thr
    370                 375                 380

cga aat gaa cat ttg cta aag caa gtg gaa gtg gac aag aca tat ccg     1200
Arg Asn Glu His Leu Leu Lys Gln Val Glu Val Asp Lys Thr Tyr Pro

385                 390                 395                 400

ctt cag gaa atg aaa aag aaa gaa gcc ctg gag ctc ttt agt tgg cat      1248
Leu Gln Glu Met Lys Lys Lys Glu Ala Leu Glu Leu Phe Ser Trp His
                405                 410                 415

gcc ttt cga aaa agt tac cct aat gaa gaa tat ctt gaa gtc tca gaa      1296
Ala Phe Arg Lys Ser Tyr Pro Asn Glu Glu Tyr Leu Glu Val Ser Glu
            420                 425                 430

aag gtt gtt tct tac tgt gga ggt ttg cca cta gca ctt gaa gtt tta      1344
Lys Val Val Ser Tyr Cys Gly Gly Leu Pro Leu Ala Leu Glu Val Leu
        435                 440                 445

ggc tct ttt ttg ttt aaa aga cct att gca gag tgg aaa agt caa ttg      1392
Gly Ser Phe Leu Phe Lys Arg Pro Ile Ala Glu Trp Lys Ser Gln Leu
    450                 455                 460

aag aaa ttg gaa aga act cct aat gga gaa ata ata aaa cca cta aaa      1440
Lys Lys Leu Glu Arg Thr Pro Asn Gly Glu Ile Ile Lys Pro Leu Lys
465                 470                 475                 480

ata agc ttt gaa ggg cta gat gat aca gag aag gct ata ttc ctt gac      1488
Ile Ser Phe Glu Gly Leu Asp Asp Thr Glu Lys Ala Ile Phe Leu Asp
                485                 490                 495

ata tct tgt ttc ttt att gga agg gac aag gac tac gtt gca aaa gta      1536
Ile Ser Cys Phe Phe Ile Gly Arg Asp Lys Asp Tyr Val Ala Lys Val
            500                 505                 510

tta gat gga tgt gga ttt ttt gca aca gga gga atc agt gtc ctc cgc      1584
Leu Asp Gly Cys Gly Phe Phe Ala Thr Gly Gly Ile Ser Val Leu Arg
        515                 520                 525

gaa cga tgt ctt gta act gtt gaa ggc aac aag ttg act atg cat gat      1632
Glu Arg Cys Leu Val Thr Val Glu Gly Asn Lys Leu Thr Met His Asp
    530                 535                 540

ttg ctt cga gaa atg gcc aga gta atc att tct gaa aaa tct ctt gat      1680
Leu Leu Arg Glu Met Ala Arg Val Ile Ile Ser Glu Lys Ser Leu Asp
545                 550                 555                 560

tgc cct gga aaa tgg agt agg ttg tgg aat cgt caa gag gtc act gat      1728
Cys Pro Gly Lys Trp Ser Arg Leu Trp Asn Arg Gln Glu Val Thr Asp
                565                 570                 575

gta ttg aca aat aaa tct gga act gga gaa att gaa gga ctt gct cta      1776
Val Leu Thr Asn Lys Ser Gly Thr Gly Glu Ile Glu Gly Leu Ala Leu
            580                 585                 590

tat ttc cct aaa gaa act aag att gct agt ttc agt aca aaa gca ttt      1824
Tyr Phe Pro Lys Glu Thr Lys Ile Ala Ser Phe Ser Thr Lys Ala Phe
        595                 600                 605

gcc ata atg aag aaa ctg agc ctg ctt gct cta ctt ttc cgt aaa gaa      1872
Ala Ile Met Lys Lys Leu Ser Leu Leu Ala Leu Leu Phe Arg Lys Glu
    610                 615                 620

act aag att gct agt ttc agt aca aaa gca ttt gcc aaa atg aag aaa      1920
Thr Lys Ile Ala Ser Phe Ser Thr Lys Ala Phe Ala Lys Met Lys Lys
625                 630                 635                 640

ctg aga ctg ctt cag ctc agc aac gta gag ctc aaa gga gaa tac aaa      1968
Leu Arg Leu Leu Gln Leu Ser Asn Val Glu Leu Lys Gly Glu Tyr Lys
                645                 650                 655

cat ctt ccc aaa gag tta ata tgg ttg cgt tgg gaa gga tgc cct tta 2016
His Leu Pro Lys Glu Leu Ile Trp Leu Arg Trp Glu Gly Cys Pro Leu
            660                 665                 670

aag tcc tta cca gat gac ttt ttt aat caa cca aga cta gtt gtt tta 2064
Lys Ser Leu Pro Asp Asp Phe Phe Asn Gln Pro Arg Leu Val Val Leu
        675                 680                 685

gag atg cag tgt agc aaa ctg gta caa gtt tgg aag ggt ttc aag tcg 2112
Glu Met Gln Cys Ser Lys Leu Val Gln Val Trp Lys Gly Phe Lys Ser
    690                 695                 700

cta cat aac ttg aaa acc ctt gat ctc agc tgg tct gat tcc tta cag 2160
Leu His Asn Leu Lys Thr Leu Asp Leu Ser Trp Ser Asp Ser Leu Gln
705                 710                 715                 720

aaa tca ccg gac ttt tca caa gtc cca aat ctt gaa gag ttg ata ttg 2208
Lys Ser Pro Asp Phe Ser Gln Val Pro Asn Leu Glu Glu Leu Ile Leu
                725                 730                 735

gaa gcc tgt att aat ctg tcc gag att cac ccc tcc att ggt cat ctt 2256
Glu Ala Cys Ile Asn Leu Ser Glu Ile His Pro Ser Ile Gly His Leu
            740                 745                 750

aaa aga ctt tct ttg gtg aac ctt aag tgg tgt aat aag ctt att tct 2304
Lys Arg Leu Ser Leu Val Asn Leu Lys Trp Cys Asn Lys Leu Ile Ser
        755                 760                 765

ctt cca agg gat ttc tat aag ttg aaa tct gtt gag act ctt ctt ctt 2352
Leu Pro Arg Asp Phe Tyr Lys Leu Lys Ser Val Glu Thr Leu Leu Leu
    770                 775                 780

aat aat tct tat caa ttc aga gaa gtg cat gag gat ata ggg gag atg 2400
Asn Asn Ser Tyr Gln Phe Arg Glu Val His Glu Asp Ile Gly Glu Met
785                 790                 795                 800

ata tca ctg aga aca ctt gaa gca agg ggt aca gcc ata aga gaa gta 2448
Ile Ser Leu Arg Thr Leu Glu Ala Arg Gly Thr Ala Ile Arg Glu Val
                805                 810                 815

cca cct tcc ata gta gga ttg aag aat ctc act cgt tta tct ctg gag 2496
Pro Pro Ser Ile Val Gly Leu Lys Asn Leu Thr Arg Leu Ser Leu Glu
            820                 825                 830

ggt act atg agg ggt att caa ttg ccc cat tcg tta aga gaa atc gat 2544
Gly Thr Met Arg Gly Ile Gln Leu Pro His Ser Leu Arg Glu Ile Asp
        835                 840                 845

ctc aca tac ggc gca tta gat gat gca att aag gat ctt ggc agt cta 2592
Leu Thr Tyr Gly Ala Leu Asp Asp Ala Ile Lys Asp Leu Gly Ser Leu
    850                 855                 860

att tat tta caa cat ttg cat ctt caa ggt aat aac ttt cat agc tta 2640
Ile Tyr Leu Gln His Leu His Leu Gln Gly Asn Asn Phe His Ser Leu
865                 870                 875                 880

cca aac ctc agt ggt ctt tta aag ctc gaa aca ttg tgg tta aat gga 2688
Pro Asn Leu Ser Gly Leu Leu Lys Leu Glu Thr Leu Trp Leu Asn Gly
                885                 890                 895

agc gaa tat ctt cgt aca atc cct gat tta cca aca aat ttg aaa gtt 2736
Ser Glu Tyr Leu Arg Thr Ile Pro Asp Leu Pro Thr Asn Leu Lys Val
            900                 905                 910

ctg ttt gcc gat gct tgc cct gca ttg gaa aca atg ccg gac ttt tcg     2784
Leu Phe Ala Asp Ala Cys Pro Ala Leu Glu Thr Met Pro Asp Phe Ser
        915                 920                 925

gaa atg tca aat atg aga aca ctg gat gta agt cat tca gcc aaa ctc     2832
Glu Met Ser Asn Met Arg Thr Leu Asp Val Ser His Ser Ala Lys Leu
    930                 935                 940

act ggg gtt cca ggc ttg gat aag tca tta aac tcc atg gtg tgg ctt     2880
Thr Gly Val Pro Gly Leu Asp Lys Ser Leu Asn Ser Met Val Trp Leu
945                 950                 955                 960

gat atg cat cgc tgc ccc aat ctc aca gct gat ttt agg aag aac atc     2928
Asp Met His Arg Cys Pro Asn Leu Thr Ala Asp Phe Arg Lys Asn Ile
                965                 970                 975

cta cag gga tgg act tct tgc gga ttt ggt ggc att gct ctc cat ggg     2976
Leu Gln Gly Trp Thr Ser Cys Gly Phe Gly Gly Ile Ala Leu His Gly
            980                 985                 990

aat tat gtt cct gat tgg ttt gag ttt atc aac gag ggc act aaa cgt     3024
Asn Tyr Val Pro Asp Trp Phe Glu Phe Ile Asn Glu Gly Thr Lys Arg
        995                 1000                1005

acg aag ttg cgg gcc tac aaa acc aaa gcc tca tat acg atc gat gag     3072
Thr Lys Leu Arg Ala Tyr Lys Thr Lys Ala Ser Tyr Thr Ile Asp Glu
    1010                1015                1020

aat tat gaa aac gat cat ctt tgg cag gga caa ttc tca aac aat gag     3120
Asn Tyr Glu Asn Asp His Leu Trp Gln Gly Gln Phe Ser Asn Asn Glu
1025                1030                1035                1040

ctc aat ttg caa ggt ggg gat aaa gtt gat ata gtt ttt gaa aat cta     3168
Leu Asn Leu Gln Gly Gly Asp Lys Val Asp Ile Val Phe Glu Asn Leu
                1045                1050                1055

gat gaa gat gat tct gtt aaa atg atg aga aca ggg gtt aat cta gta     3216
Asp Glu Asp Asp Ser Val Lys Met Met Arg Thr Gly Val Asn Leu Val
            1060                1065                1070

tgg gac aaa ctt atg aag gaa aat atg cat gat ttg ggc gac act aga     3264
Trp Asp Lys Leu Met Lys Glu Asn Met His Asp Leu Gly Asp Thr Arg
        1075                1080                1085

ggt aga atc agt ttt ggc atc tga                                     3288
Gly Arg Ile Ser Phe Gly Ile
    1090                1095

<210> 4
<211> 232
<212> PRT
<213> Malus sp.

<220>
<223> TIR domain of SEQ ID NO: 1

<400> 4
Met Thr Ala His Glu Ala Ser Ser Ser Ser Ser Ser Lys Ser Lys Leu
1               5                   10                  15
Trp Ser Tyr Asp Val Phe Leu Ser Phe Arg Gly Glu Asp Thr Arg Asn
            20                  25                  30
Gly Phe Thr Ser His Leu His Glu Ala Leu Lys Tyr Arg Gly Tyr His
        35                  40                  45
Val Phe Ile Asp Glu Asp Gly Leu Lys Arg Gly Glu Glu Ile Lys Glu
    50                  55                  60

Lys Leu Phe Arg Ala Ile Glu Glu Ser Arg Ile Ser Ile Ile Val Phe
65                  70                  75                  80
Ser Lys Met Tyr Ala Asp Ser Ser Trp Cys Leu Asp Glu Leu Val Lys
                85                  90                  95
Ile Met Glu Cys Arg Asp Lys Leu Gly Gln His Val Leu Pro Ile Phe
            100                 105                 110
Tyr His Val Asp Pro Ser His Val Arg Lys Gln Asp Gly Asp Leu Ala
        115                 120                 125
Glu Ala Phe Gln Lys His Glu Lys Asp Ile Arg Glu Glu Lys Asp Asp
    130                 135                 140
Glu Lys Arg Glu Ala Lys Gln Lys Arg Ala Glu Gln Trp Lys Gly Ala
145                 150                 155                 160
Leu Thr Lys Ala Ala Asp Leu Ser Gly His His Leu Lys Ser Thr His
                165                 170                 175
Asn Arg Cys Glu Ala Lys Phe Ile Lys Lys Ile Val Asp Glu Asn Ile
            180                 185                 190
Trp Glu Trp Leu Pro Arg Thr Asn Glu Leu His Val Ala Lys His Pro
        195                 200                 205
Ile Gly Ile Lys Ser Arg Ile Gln Gly Ile Ile Ser Asp Leu Ser Ser
    210                 215                 220
Gly Gly Ser Asn Asp Val Leu Met
225                 230

<210> 5
<211> 23
<212> PRT
<213> Malus sp.

<220>
<223> Conserved region of TIR domain

<400> 5
Asp Val Phe Leu Ser Phe Arg Gly Glu Asp Thr Arg Asn Gly Phe Thr
1               5                   10                  15
Ser His Leu His Glu Ala Leu
            20

<210> 6
<211> 39
<212> PRT
<213> Malus sp.

<220>
<223> Conserved region of TIR domain

<400> 6
Ile Lys Glu Lys Leu Phe Arg Ala Ile Glu Glu Ser Arg Ile Ser Ile
1               5                   10                  15
Ile Val Phe Ser Lys Met Tyr Ala Asp Ser Ser Trp Cys Leu Asp Glu
            20                  25                  30
Leu Val Lys Ile Met Glu Cys
        35

<210> 7
<211> 21
<212> PRT
<213> Malus sp.

<220>
<223> Conserved region of TIR domain

<400> 7
Lys Leu Gly Gln His Val Leu Pro Ile Phe Tyr His Val Asp Pro Ser
1               5                   10                  15
His Val Arg Lys Gln

            20

<210> 8
<211> 16
<212> PRT
<213> Malus sp.

<220>
<223> conserved region of TIR domain

<400> 8
Trp Lys Gly Ala Leu Thr Lys Ala Ala Asp Leu Ser Gly His His Leu
1               5                   10                  15

<210> 9
<211> 29
<212> PRT
<213> Malus sp.

<220>
<223> Conserved motif of TIR domain

<400> 9
Arg Thr Asn Glu Leu His Val Ala Lys His Pro Ile Gly Ile Lys Ser
1               5                   10                  15
Arg Ile Gln Gly Ile Ile Ser Asp Leu Ser Ser Gly Gly
            20                  25

<210> 10
<211> 312
<212> PRT
<213> Malus sp.

<220>
<223> NBS domain of SEQ ID NO:1

<400> 10
Val Gly Ile Trp Gly Met Gly Gly Leu Gly Lys Thr Thr Val Ala Lys
1               5                   10                  15
Ala Ile Tyr Asn Gln Ile His Arg Lys Phe Glu Phe Lys Ser Phe Leu
            20                  25                  30
Ala Asp Val Ser Asp Ala Thr Ser Lys His Gly Leu Val Tyr Leu Gln
        35                  40                  45
Glu Lys Leu Ile Ser Asp Ile Leu Lys Gln Lys Ser Gly Ser Ser Asn
    50                  55                  60
Phe Asp Glu Gly Ile Asn Leu Ile Lys Gln His Leu Gln Arg Arg Arg
65                  70                  75                  80
Val Leu Val Ile Ile Asp Asn Ile Asp Asp Leu Lys Gln Leu Asp Ala
                85                  90                  95
Ile Ala Val Ser His His Cys Phe Gly Pro Gly Ser Arg Ile Ile Ile
            100                 105                 110
Thr Thr Arg Asn Glu His Leu Leu Lys Gln Val Glu Val Asp Lys Thr
        115                 120                 125
Tyr Pro Leu Gln Glu Met Lys Lys Lys Glu Ala Leu Glu Leu Phe Ser
    130                 135                 140
Trp His Ala Phe Arg Lys Ser Tyr Pro Asn Glu Glu Tyr Leu Glu Val
145                 150                 155                 160
Ser Glu Lys Val Val Ser Tyr Cys Gly Gly Leu Pro Leu Ala Leu Glu
                165                 170                 175
Val Leu Gly Ser Phe Leu Phe Lys Arg Pro Ile Ala Glu Trp Lys Ser
            180                 185                 190
Gln Leu Lys Lys Leu Glu Arg Thr Pro Asn Gly Glu Ile Ile Lys Pro
        195                 200                 205

Leu Lys Ile Ser Phe Glu Gly Leu Asp Asp Thr Glu Lys Ala Ile Phe
    210                 215                 220
Leu Asp Ile Ser Cys Phe Phe Ile Gly Arg Asp Lys Asp Tyr Val Ala
225                 230                 235                 240
Lys Val Leu Asp Gly Cys Gly Phe Phe Ala Thr Gly Gly Ile Ser Val
                245                 250                 255
Leu Arg Glu Arg Cys Leu Val Thr Val Glu Gly Asn Lys Leu Thr Met
            260                 265                 270
His Asp Leu Leu Arg Glu Met Ala Arg Val Ile Ile Ser Glu Lys Ser
        275                 280                 285
Leu Asp Cys Pro Gly Lys Trp Ser Arg Leu Trp Asn Arg Gln Glu Val
    290                 295                 300
Thr Asp Val Leu Thr Asn Lys Ser
305                 310

<210> 11
<211> 19
<212> PRT
<213> Malus sp.

<220>
<223> Conserved region of NBS domain

<400> 11
Val Gly Ile Trp Gly Met Gly Gly Leu Gly Lys Thr Thr Val Ala Lys
1               5                   10                  15
Ala Ile Tyr


<210> 12
<211> 29
<212> PRT
<213> Malus sp.

<220>
<223> Conserved region of NBS domain

<400> 12
Lys His Gly Leu Val Tyr Leu Gln Glu Lys Leu Ile Ser Asp Ile Leu
1               5                   10                  15
Lys Gln Lys Ser Gly Ser Ser Asn Phe Asp Glu Gly Ile
            20                  25

<210> 13
<211> 15
<212> PRT
<213> Malus sp.

<220>
<223> Conserved region of NBS domain

<400> 13
His Leu Gln Arg Arg Arg Val Leu Val Ile Ile Asp Asn Ile Asp
1               5                   10                  15

<210> 14
<211> 25
<212> PRT
<213> Malus sp.

<220>
<223> Conserved region of NBS domain

<400> 14
Gln Leu Asp Ala Ile Ala Val Ser His His Cys Phe Gly Pro Gly Ser

1                   5                   10                  15
Arg Ile Ile Ile Thr Thr Arg Asn Glu
            20                  25

<210> 15
<211> 28
<212> PRT
<213> Malus sp.

<220>
<223> Conserved region of NBS domain

<400> 15
Lys Thr Tyr Pro Leu Gln Glu Met Lys Lys Lys Glu Ala Leu Glu Leu
1               5                   10                  15
Phe Ser Trp His Ala Phe Arg Lys Ser Tyr Pro Asn
            20                  25

<210> 16
<211> 16
<212> PRT
<213> Malus sp.

<220>
<223> Conserved region of NBS domain

<400> 16
Lys Val Val Ser Tyr Cys Gly Gly Leu Pro Leu Ala Leu Glu Val Leu
1               5                   10                  15

<210> 17
<211> 15
<212> PRT
<213> Malus sp.

<220>
<223> Conserved region of NBS domain

<400> 17
Thr Glu Lys Ala Ile Phe Leu Asp Ile Ser Cys Phe Phe Ile Gly
1               5                   10                  15

<210> 18
<211> 13
<212> PRT
<213> Malus sp.

<220>
<223> Conserved region of NBS domain

<400> 18
Met His Asp Leu Leu Arg Glu Met Ala Arg Val Ile Ile
1               5                   10

<210> 19
<211> 24
<212> PRT
<213> Malus sp.

<220>
<223> region with LRR motif

<400> 19

Met Lys Lys Leu Ser Leu Leu Ala Leu Leu Phe Arg Lys Glu Thr Lys
1               5                   10                  15
Ile Ala Ser Phe Ser Thr Lys Ala
            20

<210> 20
<211> 24
<212> PRT
<213> Malus sp.

<220>
<223> region with LRR motif

<400> 20
Phe Ala Lys Met Lys Lys Leu Arg Leu Leu Gln Leu Ser Asn Val Glu
1               5                   10                  15
Leu Lys Gly Glu Tyr Lys His Leu
            20

<210> 21
<211> 23
<212> PRT
<213> Malus sp.

<220>
<223> Region with LRR motif

<400> 21
Pro Lys Glu Leu Ile Trp Leu Arg Trp Glu Gly Cys Pro Leu Lys Ser
1               5                   10                  15
Leu Pro Asp Asp Phe Phe Asn
            20

<210> 22
<211> 23
<212> PRT
<213> Malus sp.

<220>
<223> Region with LRR motif

<400> 22
Gln Pro Arg Leu Val Val Leu Glu Met Gln Cys Ser Lys Leu Val Gln
1               5                   10                  15
Val Trp Lys Gly Phe Lys Ser
            20

<210> 23
<211> 23
<212> PRT
<213> Malus sp.

<220>
<223> Region with LRR motif

<400> 23
Leu His Asn Leu Lys Thr Leu Asp Leu Ser Trp Ser Asp Ser Leu Gln
1               5                   10                  15
Lys Ser Pro Asp Phe Ser Gln
            20

<210> 24
<211> 24
<212> PRT
<213> Malus sp.

<220>
<223> Region with LRR motif

<400> 24
Val Pro Asn Leu Glu Glu Leu Ile Leu Glu Ala Cys Ile Asn Leu Ser
1               5                   10                  15
Glu Ile His Pro Ser Ile Gly His
            20

<210> 25
<211> 24
<212> PRT
<213> Malus sp.

<220>
<223> Region with LRR motif

<400> 25
Leu Lys Arg Leu Ser Leu Val Asn Leu Lys Trp Cys Asn Lys Leu Ile
1               5                   10                  15
Ser Leu Pro Arg Asp Phe Tyr Lys
            20

<210> 26
<211> 23
<212> PRT
<213> Malus sp.

<220>
<223> Region with LRR motif

<400> 26
Leu Lys Ser Val Glu Leu Leu Leu Asn Asn Ser Tyr Gln Phe Arg Glu
1               5                   10                  15
Val His Glu Asp Ile Gly Glu
            20

<210> 27
<211> 23
<212> PRT
<213> Malus sp.

<220>
<223> Region with LRR motif

<400> 27
Met Ile Ser Leu Arg Thr Leu Glu Ala Arg Gly Thr Ala Ile Arg Glu
1               5                   10                  15
Val Pro Pro Ser Ile Val Gly
            20

<210> 28
<211> 41
<212> PRT
<213> Malus sp.

<220>
<223> Region with LRR motif

<400> 28
Leu Lys Asn Leu Thr Arg Leu Ser Leu Glu Gly Thr Met Arg Gly Ile
1               5                   10                  15
Gln Leu Pro His Ser Leu Arg Glu Ile Asp Leu Thr Tyr Gly Ala Leu
            20                  25                  30

Asp Asp Ala Ile Lys Asp Leu Gly Ser
        35                  40

<210> 29
<211> 22
<212> PRT
<213> Malus sp.

<220>
<223> Region with LRR motif

<400> 29
Leu Ile Tyr Leu Gln His Leu His Leu Gln Gly Asn Asn Phe His Ser
1               5                   10                  15
Leu Pro Asn Leu Ser Gly
            20

<210> 30
<211> 21
<212> PRT
<213> Malus sp.

<220>
<223> Region with LRR motif

<400> 30
Leu Leu Lys Leu Glu Thr Leu Trp Leu Asn Gly Ser Glu Tyr Leu Arg
1               5                   10                  15
Thr Ile Pro Asp Leu
            20

<210> 31
<211> 23
<212> PRT
<213> Malus sp.

<220>
<223> Region with LRR motif

<400> 31
Pro Thr Asn Leu Lys Val Leu Phe Ala Asp Ala Cys Pro Ala Leu Glu
1               5                   10                  15
Thr Met Pro Asp Phe Ser Glu
            20

<210> 32
<211> 24
<212> PRT
<213> Malus sp.

<220>
<223> Region with LRR motif

<400> 32
Met Ser Asn Met Arg Thr Leu Asp Val Ser His Ser Ala Lys Leu Thr
1               5                   10                  15
Gly Val Pro Gly Leu Asp Lys Ser
            20

<210> 33
<211> 23
<212> PRT
<213> Malus sp.

<220>

<223> Region with LRR motif

<400> 33
Leu Asn Ser Met Val Trp Leu Asp Met His Arg Cys Pro Asn Leu Thr
1               5                   10                  15
Ala Asp Phe Arg Lys Asn Ile
            20

<210> 34
<211> 28
<212> PRT
<213> Malus sp.

<220>
<223> Connecting region

<400> 34
Gly Thr Gly Glu Ile Glu Gly Leu Ala Leu Tyr Phe Pro Lys Glu Thr
1               5                   10                  15
Lys Ile Ala Ser Phe Ser Thr Lys Ala Phe Ala Ile
            20                  25

<210> 35
<211> 119
<212> PRT
<213> Malus sp.

<220>
<223> C-terminal region

<400> 35
Leu Gln Gly Trp Thr Ser Cys Gly Phe Gly Gly Ile Ala Leu His Gly
1               5                   10                  15
Asn Tyr Val Pro Asp Trp Phe Glu Phe Ile Asn Glu Gly Thr Lys Arg
            20                  25                  30
Thr Lys Leu Arg Ala Tyr Lys Thr Lys Ala Ser Tyr Thr Ile Asp Glu
        35                  40                  45
Asn Tyr Glu Asn Asp His Leu Trp Gln Gly Gln Phe Ser Asn Asn Glu
    50                  55                  60
Leu Asn Leu Gln Gly Gly Asp Lys Val Asp Ile Val Phe Glu Asn Leu
65                  70                  75                  80
Asp Glu Asp Asp Ser Val Lys Met Met Arg Thr Gly Val Asn Leu Val
                85                  90                  95
Trp Asp Lys Leu Met Lys Glu Asn Met His Asp Leu Gly Asp Thr Arg
            100                 105                 110
Gly Arg Ile Ser Phe Gly Ile
        115

<210> 36
<211> 9780
<212> DNA
<213> Malus sp.

<220>
<221> source
<222> 1..9780
<223> /mol_type="unassigned DNA"
      /note="Vr2-A gene "
      /organism="Malus sp."

<400> 36
ttgccggaga tgagagagag gatagcaaga agagagatga gcggcgggag tgtgggagag       60

gccgaacaat tttagagttt tcagtttatt attattattt tttaatgtga tatatataaa      120

ataaactggt aaatagatat ctgttataat taggagcaat aatcataatt atccatttaa 180

attttacgga taaataattg tatccataac tttttattta tttaaatgat tatcaataat 240

cataatcgtg aattttaaat gaacggggta atagtacccg gggaggaggg gggtattttg 300

ctcattccaa atctcaactc gccacttcct ggaaagcaaa gagaaagcca attggtggaa 360

tattgaattg ggatgtaatc atgtgagtgc agtagacgta ggcgttcttg tatgtatttg 420

agtgcttctg gcattgctcc atccacattt tccttgcctt gcccgtcaca gggcaaaact 480

tatcactcta accctacttt ctctaccttc aatgtttcta tacatgaaaa tcgatctggt 540

ccgttctata cttgacagga aaaataattc tatacaaatt tcaatgcact tgacactcac 600

ttcaggatca atggcattga ttggataagg tgagcaaatt cttatcaata ccttccctca 660

ttgtttagtt tttttataaa acatcataac tccaaaggaa gccataaagc atcataactc 720

caaaccaaaa accagtttta tttataatat atttagactt tggtggcttg ttggtatagt 780

gttttttaca gtaattagga ctaatataaa ttaaaagctc atctggtaac gtttcactac 840

cagtttaaaa atcaaaacaa caaaaataga actttacatg tttaattaaa atctcaaaac 900

gacaaagttg catgactttg actccgcgct agtttatatg tatgtgaaga ttattatgct 960

aaaatggatt ctcagtgtgc aactctccgt ttctggaact cggtcatttc tcaagtttta 1020

gatgtgaagg ctaattatta agactttaga cttgggtatt tatttcggta ttatctttgt 1080

agcttaccac acacacataa caaatgcaaa tattgtaaag attcatacag actgatcaat 1140

gacccatcat taatcacacc aattatgaca tcccacatcg cccaggggag tgatccttaa 1200

atgtatattc tcattcctac ctagcacgag gcattttggg agctcactgg cttcgggttc 1260

cgtagaaact cggaagttaa gcaagaagga ggtcagatca ctcccaggat gggtgaccca 1320

ctgggaagtt gctcatgagt tcccaaaaac aaaaccgtga gggaatggta agcccaaagc 1380

ggacaatatc gtgttacggt ggttgagcgg gcctgtgatg tgacaaattg gtatcagagc 1440

caatccctgg ccggaagtgt gccgacgagg acgtcgggcc cctaatgggg gtggattgtg 1500

acatcccaca tcgcccagga gagtgatcct taaatgtata ttctcatccc tacctagcat 1560

gatgcttttt gggagctcac tggcttcgta ttccgtagga actccgaagt taagcgagaa 1620

ggatgccaga gcactcccag gatgggtgat ccactgggaa gttgctcatg agttcccaaa 1680

aacaaaaccg tgagggaatg gtaagcccaa aacggacaat atcgtgctac ggtggttgag 1740

caggcccggg atgtgatgga cccgggccgg gatgtgacac caatactttc tctcaccctt 1800

aatcttacca catgcacaac atgtcatacg tgaaagtttt atcacaaaat actttgatat 1860

aattaaaagt ggaaaccaca caatataagt tatctttaat tttgtgaaat tttatatgag 1920

atttttgtac acttcaacat gtatagggat ggacttcttg tggatatggt ggcatttgtc 1980

tcatggtatt tgtgatattc ccgagtagtt tgagtttgct tatgagggaa atgtaactga 2040

ctttattata cctgaaagtg atgtttaaag ggttggttgt gcatttactc gtcaagtgaa 2100

caacttgaca gtcctcatgt ggttattgtt ataaattcta ccaagcatac cacttggcac 2160

gccttgatag catttacctc agtactacca aaatctcagg aaagcgaaag cgattatctt 2220

tggcaaggag agatattgta gaaaaaaaaa aaacagtgtg tgacaaaaat taatgcataa 2280

gaagcaaaat taaaacagaa tgttactaac attggctggg aataaatgta acatgaggta 2340

gatcttaatt aatgtaacgg gaacatcaat gatataccat gtgtttttag gtaagtgatg 2400

agaaatttta ttttttaagt tattaaattt ttaaaacata ttctactatt tgtataataa 2460

tacttgatgt atcacatcgt gtgtcagtca cactgaaaaa tctctctttc acgttagcct 2520

tttccttgtg ttcagatatt cttgaaaagc aaacacgaac cttttccttc acgtgaataa 2580

agcatctttc agaattcaga gttcctttcc tattacatgt cggtagtatg tccttttgtg 2640

ttcttttctt tgatcttttc ctctctcttt ccttacacct tcccgccggg tagtgagtcg 2700

tttctcatgt ccatacgcca acattattat attataagct tttgcttttg tttactgaag 2760

gatcttaact tgtgtttagg tatcatcctt cggcaacaca aacacaaccc ttgccatttt 2820

tcagtctctg atctgaacca ctgcagtgcc ataacggtgg ataccgccat gacaggccat 2880

gaagcctcct cttcttcctc ctccaagtca aaactttgga attacgacgt gttcttgagc 2940

ttcagaggcg aagacacgcg caagggcttc acaggccatc tccacgcggc attgaaagac 3000

aggggatacc aggcttatat ggatgaggac gatctaaaca gaggggaaga aataaaagag 3060

gaactgttcc gggcaatcga agggtcgagg atctctatca ttgtcttctc aaaaggatat 3120

gcggattcga gttggtgtct tgacgagcta gtgaagataa tggagtgcag atccaaactg 3180

aggcgacatg ttttgccaat attctatcat gttgatcctt cacatgtcag gaagcaggac 3240

ggagatttag ccaaagcatt tcagaagcac aaaaagggca tcagtaaact aaaagatgcc 3300

aagaaacgtg aagcgaaacg agaaagggta aagcagtgga gaaatgctct cacggaagcc 3360

gcaaatttgt ccggccacca tcttcaaatc actgaaaatg ggtaatcatt cacctgcttt 3420

tcaagatttc aaacatttta gaggacaatt gttagaagag ggagtatcac acacaaatga 3480

aaaaatatat acgaatttat aaaatcaaga aatagatctg aaatttagac atctttaagt 3540

tgtattactc ttattaactt gctagctaat aatttaacca aacaacttat aatttaactt 3600

gctagctacc ttctttatgg aaatttttta gttgtggcgg gaacattgat gatacaccac 3660

gtgttttcag gtaagtagtg agaaatttta ttttttaagt tattaacttt ttaacacaca 3720

tatctcacga tttatatagc tacacatgat ataccacctt atatgacgat cacactgaaa 3780

aatctctcat ttatttgtct ttactctttt ttcacttgta cgggttaccg taccaaaacc 3840

cttataccaa ttatcgtacc aaactttcaa tttggtaaaa tctattatca ttaccatacc 3900

aaactttcgg tatatcaaag tttggtattg ccaaaagttc ggttgacatg gtatggcaat 3960

ggtaattgct attttgtttt gggacaaaat ctgtttttgt ttttttaacc caattcaagg 4020

gcaaaacttt ttttttgtac ctttatctca tacattatag attaaattca tcattcacaa 4080

ttcacacata taataattca aatgatgcat caagattcat catgaaaatt aagtttacaa 4140

tccaaataga agttacgaat aaaaacaaat agaagttaac aatccaaata gaaattaaag 4200

tttcaaacca aatgaaaatt ggaagtaaac ttcaaaaatg agaattcatt gatcaggtat 4260

tcaaacttga gatgtcaaag tttttggata aggcattgaa cttatagaag attgagtttg 4320

tgtcaagcct aaattacaaa caaagaaaac atattaatta gtagcaagaa taattaaagt 4380

tgaaaaccat ttaataacaa atttactgaa aaaattaaag catatctttc ttatttcctc 4440

ttcttctatt tccttgtaaa attgaagcat atctttcgtt gattccttgt agaagtttac 4500

ttcatctgcc ctaagccaat caatagtacg cactagtgcc tccattattt taggagttaa 4560

ggatacccta aaagggtcca caacctcatc cttaggctaa atgcattttc actagcaaca 4620

atagaagtga gggttacaaa gatattttgg ctatttgtga aagaattagg aactcttctt 4680

gtttgatttc cacaatttca agagatcaaa gttaacaaca acaatgctaa tataagtagg 4740

gttttatttt caaattattg gaatattata aatatgtgtt atataattat ctattatata 4800

atttataaat gatatattat attgtatttt cggtatgata cggtaatacc gtggtaatgg 4860

tattcattac caataccgta ccatgaaatt ttggtacgat acaataccgt acccttaccg 4920

attggtacaa aaaatttggc acaaaatcgg tatggtacgg ttggcaattc ggttggcacg 4980

gtaatttggc aaaaaaatcc accctctttc ccactccttt catgccatgt ggaatcaact 5040

tatacttata tgtatttaat gtgtatagaa atttatttct ctttccaaat acgcccttaa 5100

ttaatgtttc agatttttgt tttcttgagt gcaaatgttt catattttaa ccaaactatt 5160

attacttatc ttttttatta aaaaatttta agcatttctt atttgctttg ccatcttctg 5220

agaaatttta atctccacaa aaacaatata ggaggtgact gcctactatt tataaatatt 5280

caaacattat aatttatatc caacagaaaa aaaaattata atttataagc ctccggcttt 5340

tatataatac aagagaaaga tgcattgaaa taccaatttt gtgaaattca tgtaaaaaaa 5400

attgcgcaaa ttgttataca aataatgcaa ttaaacagag ttaaacaatt caaaaagaaa 5460

aacaattaaa aaaatcttat attagcattt tattttccac tttgaattcc catttgtctt 5520

tttcttttct tgtaccaatt ctcggttaac tttgtcctcc tgtactgttt ttagtgggaa 5580

aaaatttcca ggaacagaat aatgcatttg aaaaaaagaa cggcaaaaga aaataaaatt 5640

gaaagacaac tccacccaga gcttcagaac ataatttatt taataaaaaa atccataaca 5700

aattagaaat gtttaaaaag ctatatttta ataaaaaaaa gtgaagcaat aattgtttgg 5760

ttaaactctg gaacattaaa ttaggggcat attaagaaag aaaaaaaata aaattctcta 5820

ccaattaaat acataaatgc atatgtaagt tgattccaca gtaagtaaat aaagaaagag 5880

aattgtttat gttctgatat ctcctacata gcctttcatt gtgtcccact aatgcttaag 5940

aattcaattt tcaggagcga agcggacttc attaggatta ttgttgatga gaatatttgt 6000

gaatggctca ccagcacaaa cgaattacat gtggccaatt acccagttgg aatcgattct 6060

cgcattcaag atatcatcac ttatctttca agtggtggat caaatgatgt tcgcatggtt 6120

ggaatttggg ggatgggtgg agtgggtaaa acaacagttg ccaaagccat ttataaccaa 6180

attcatccta tgttccaatt caaaagtttc cttgccgacg ttcgtgatgc tacaagtaaa 6240

catggtctgg ttgatttgca aaacaaactt atttctgaca tcttgaaaaa gaagcctgaa 6300

ataagatgtg ttgatgaagg tatcgttatg ataaaacaac aatttcgaca taaaagggta 6360

cttgtcatca tggacaacat agacgaagta gaacaattgg atgcaatagt tggaaatcat 6420

gattggtttg gtccagggag tagaattatc ctaacaacaa gagatgaaca tttactaaag 6480

cgagggaaag tgcacaacat atatccggct cagaaattca atgaaggaga agctctggag 6540

ctctttagtt ggcatgcctt tggaaatggt tgtccaaaca aaggatatca tgaactctca 6600

aaaaaagttg tttcttattg tggaggtttg ccactagccc ttaaagtttt aggttcattt 6660

ttgtttgaaa gaaccatggc agagtggaaa agtcaattgg agaaattgga aagaactccc 6720

gatggaaaaa taataaaacc actaagaata agctttgatg ggctagatga taaacaaaag 6780

gctatattcc ttgacatatc ttgtttcttt attggaatgg acaaggacaa tgtcgcaaaa 6840

gcattagatg tatgtggatt ttctgcgaca atagaaatta gtattcttcg tgaacgatgc 6900

cttgtaactg ttgaagataa gaagttgaat gtgcatgatt tgcttcgaga aatggccaaa 6960

gtaatcattt ctgaaaaatc ccctagtcac cctgaaaaat ggagtaggtt gtggaaccat 7020

caagaggtcg tagatgtctt gagaaataaa tctgtaagta cattcccaat aaaattttgc 7080

attaatgcat cctacatata acccacgtct atctgttatg cactaaacaa gatttatgtg 7140

taacactaaa tatattcacc atagttgatt tagcctaatg aataataata tgcaaataat 7200

ctgtttctta cacatgaata taaccttttg ttaacaggga actgaagaag ttgaaggact 7260

tgctctacat aaaccttttt ctcatgacaa ttctagtttc aatacagaag catttgccaa 7320

tatgaagaaa ctgagattgc tcctgctata caaggtagag cttaatggag aatacaaaca 7380

tcttcccaat gagttaatgt ggttgcgttg ggaagaatgc cttttaaagt ccataccaga 7440

tgactttttc aatcagccaa gactagttgt tttagagatg cagcggagct atctggtaca 7500

agtttgggaa ggttccaagg tactcgatta cacatgatga tgttctattt ctatttgttt 7560

ttggatttcc ttctttttat gagatgttcc ttttgaatat taatttcaaa ttttctttgg 7620

ttttgtgcaa cagtcgcttc agaatttgaa aaatcattga tctcactcgg tcctattcct 7680

taataaaatc accagacttt tcacaagtcc caaatcttga agagttgata ttggaaggtt 7740

gtgagagttt gtatgagatt gattcctcca ttggtcatct taaaagactt tctttggtga 7800

accttagagg atgcagaatg cttacttctc ttccaaggga tttctataag tccaaatctg 7860

ttgagactct ttgtcttaat gactgttcag aattcagaga agtgcatgag gatttagggg 7920

agatgatatc attgagaata cttgaagcag atttcacagc cataagacaa ataccaactt 7980

ccatagtaag attgaagaat ctcactcgtt tatccctaat aaatcctatt tcccgaagag 8040

gttcatccct aataggtgtg gaaggtattc atttgcccaa ttcattaaga gaattaagtc 8100

tctcggtgtg caaattagat gatgatgcaa ttaagaatct tgggagtcta atttctttac 8160

aatatttgga tcttggatgg aataaatttc atacccttcc tagcctcaat ggtctttcaa 8220

agcttgaaac tttgcagtta agtggatgca tgtatcttca tacaatccct gatttaccaa 8280

caaatttgaa ggttctgcat gtcgatgaat gccctgcgtt ggaaacaatg cccaattttt 8340

cggaaatgtc aaatataaga caactccatg taagtcattc acccaaactc actgaggttc 8400

caagcttgga taagtcatta aactccatga tatggattga tatgcatgag tgcaccaatc 8460

tcacagctga ttttaggaag aacatcctac aggtatatat atctctctct ctcccattgc 8520

cttcatatcg tgcacatgca cacacaaacg cacatgtgca tgtatatata tttatgacac 8580

ttgcattgta tgtgtaggga tggacttctt gcggatttgg tggcattgcc ctccatggga 8640

attatgttcc tgattggttt gagtttgtca acgagggcgc taaagttagt tttgatattc 8700

ccccgactca tgatcgtact tttgaagggc taactctgtt cttcttgtac cgcccattca 8760

aagcgacaat tttaaatcct cttgaaatca ctgttataaa taataccatg tgtacagtgt 8820

tgcgggccta cgtaaacaac agaggttctc tgattaatac atttgtagat ggtgatgaat 8880

atctttggca agtacaactc tcgaacaatg agctcaattt gcaaggtggg gataaagttg 8940

atatactttt taaagacgag tactatgcgc ggaataatgc tcttaaaatg atgagaatag 9000

gggttaatct agtatgggac aaacctatga agaaaaatat gcatgatttg gacagagcta 9060

gattttcttg tcactggaga tccagcttac ttcttggatg agggaggacc aagccatgat 9120

gcatctgata aactcgtttg tgcagaatga aggtggtgac agcatcacag tcaattcgtc 9180

aaacgcctcg tcatttttcg ctttcctagt tcgataatgg aggcaagtcc atatttcata 9240

ccactttttc agattcctac gattttttga ataagtagtt ttgagttttg aagatatatt 9300

gcatcaagaa acatttcaac attacaacaa tcaataatca atagaccacc caaatatttg 9360

acaaatacta ctcaaatact tgccctgttt ccgccacata ttcaaggaat ttactgttag 9420

actatgtttg gacgacggaa ataaacttga aattgagata aaaatcagaa tttataaatt 9480

ggcatgcatc aattcccttg tttggattca taaacataga aatttagaat tttcttgtgg 9540

aataaaaaaa cttggaattt ggaaccttca atttccaagt ttaaattcca tataaataag 9600

tgtcatttcc caatatctat gattgagagt ttaaaaatat caaattacgt attcaattct 9660

attgtttttt taggttaatc aaacaagaaa attcacaaat tttaaaaaat aaaatactat 9720

aattttaaaa ttcataagta atcttaaatt tcttcatcca cacatagtgt taaggttaga 9780

<210> 37
<211> 8514

<212> DNA
<213> Malus sp.

<220>
<221> source
<222> 1..8514
<223> /mol_type="unassigned DNA"
      /note="Vr2-B gene"
      /organism="Malus sp."

<400> 37
attcctccag cccaccaaac acagtactaa tatatacata tatatgcagt tatatatttg      60

tatatgcacc ttctttagtt tctttgtcaa ttatataatc tctctgttaa tattaaaaaa     120

catttgattt agtaacttga tctcaactat tgtaatgcag ttcaagattt ccacgttaat     180

ttagtttaat attaaataaa tggtttgcac agctaattgc atgtagttct ctttatctct     240

attctcctca tcaatgcgaa ggaatgtaaa gatttttttt ctcccatttt cattatgccc     300

aatttcaaat tgaaatatat ttatttatct ttcttttgaa taggacttcg acctactaat     360

tttttcctga aattatagcc tcttaatcga tagtaaaaaa taaatggtga atataagaac     420

aaactcattt gtgctaattt tttaatgctt aataagattt acaaaaccct gtattttaaa     480

aaatagttat tttttagttt tgtggactat cctctcttac cgattgtcaa caaccttcca     540

tgggaccttc gattcccaag gagttcaata aattatagac tacaatgcaa gtaaaaaatt     600

aatctaaaag ctttaaaaat tttgaacatt tgataatttg ctttccaaaa ttctgattat     660

ggctgtactt ttttcaatcc ttacattctt tattacgttc ttaaattatt ttagtacaat     720

gtgttaaata tttctgatta ctaaatcaaa tcatcaccat tcaatttgac aggaaaaaga     780

atcctaacat tattcttatg attcacatat gtgtgattct caatgcactt gacagaaaaa     840

agatttctat acaaatttca atacacttga cactctcttc agggtcattg gcattgattg     900

gataaggtga gcaatttctt atcaatgctt tctctcattg tttagtttat tttttttata     960

aaacatcata actccaaacg aagccataaa gcatcataac tccaaaccaa aaaccagttt    1020

tatttataat ataacattta gactttggtg gcttgttggt atagtgtttt tacagtaatt    1080

aggactaata taaataaaaa gctcatctgg taacgtttta ctaccagttt aaaaatcaaa    1140

acaacaaaaa tagaacttta catgtttaat taaaatctca aaaagacaaa gttgcatgac    1200

tttgactctg cgctagttta tatgtacgtg aagattatta tactaaaatg gattgtcttt    1260

gcaacttcaa ttttgtgcaa ctctccgttc ccgggattcg gtcatatctc aagtttttga    1320

cgtgaaggct atattattaa atttagactt gggtatttat ttcggtatta tctttggtag    1380

tttaccacac gcacataaca aatgcaaata ttgtcaaagg ttcatacata ctgatcaacg    1440

acccatcatt aatcacacca atattttctc tcagcttgaa tcttaccacg tgcacaacat    1500

atcatatgtg aaagttttat cataaaatac tttggtataa ttagaagtgg aaaccgcaca    1560

atataagtta tctttaattt tgtcaaattt tatgtgagat tcttgtacac ttcaacatgt    1620

attgggatgg actccttgtg gaaatggtgg tatttatctc atggtattta tgatattccc 1680

gagtggtttg agttcgctta tgagggaaat gtagctgact ttgttatacc tgaaagtgat 1740

ggttgtaatt ttaaagagtt ggttgtctga cgcatttcct cgtcaagtga acaacttgaa 1800

ggtcctcatg aggttattgt tataaattcg accaagcata ctactttgca caccttgata 1860

acatttacct cagtgctacc aaaatctcat gaacgcgaaa ccgattatct ttggcaagga 1920

gagatactgc agaataaaaa catagtgcga aaaaaattaa tgtatatgtt cttgaagaaa 1980

attaatgtat aaaaagcaaa attaaaacag aatgttacta acattggttg ggaataaatt 2040

gtaacatgag gtagatctta attaaacagg agccttttcc ttgtgttcag gtattcttga 2100

agaaaagcaa acatgagcct tttccttcac gtgaataaag catatttcac tttcagagtt 2160

ccttccttcc ctgctttcca ccgtcctatt acgtttcggt agtctgtcct ttcgtgtttc 2220

tttatttccc cttttcctct ctctttcttt gcaccttcct gcccggtagt gtgtcgtttc 2280

tcaagtccat acgccaacat tattatatta taaacctttt gcttttgttt actgaaggat 2340

cttgacttgt gttaaggtat catccttcgg cgacacaacc acaaccattg ccattttttc 2400

agtctctgat ctgaaccatt gcagtgccat aacggtggat accgccatga cagcccacga 2460

agcctcctct tcgtcctcct ccaagtcaaa actttggagt tacgacgtgt tcttaagctt 2520

cagaggcgaa gacacgcgca atggcttcac tagccacctc cacgaggcat taaaaaacag 2580

aggataccat gtctttattg atgaggacgg tctaaaaaga ggggaagaaa taaaagagaa 2640

actgtttcgt gcaatcgaag agtcgaggat ctctatcatt gtcttctcaa ggaggtatgc 2700

ggattcgagt tggtgtcttg acgagctggt gaagatcatg gagtgcagag gcaaactggg 2760

gcatgttttg ccaatattct atcacgttga tccttcgcat gtcaggaagc aggacggaga 2820

tttagctgaa gcatttcaga aacacgaaac ggacatctgt gaagaaaaag atgacgagaa 2880

acgtaaagct aaacaagaaa gggtaaagca gtggagagag gctctcacag aagctgcaaa 2940

tttgtctggc caccaccttc aaagcgctaa taataggtaa ttattcacct acttgtcaag 3000

atttaagttg taatactcta attaacatac ttaattaata caccttaatc tctcacgctg 3060

atccttcaca tgtcaggaag aggcggagag agggtcatac agaagataca aatttgtctg 3120

accaccaaac aactggcaac gggtaatcat ttacctgctt atttcaagat ttgaaacatt 3180

ttagtgaatg acttgatctc tcaattaatt aacattttac ataatttttt tttttttttt 3240

gtagaaaagc tcgacggatc taaactttag tttacatgct taatgaatac accttaatct 3300

ctcatataaa acctaaaccg taattgtagt ttaattcaca cacctaacta ctttctttct 3360

tttattcaag tttcaagtta tcgtcacaaa atcacatgct agctgctagt tagtgtatta 3420

tatatatata tatatatata tatatatgaa gaaaagaaag aaaattgtca tgtactgata 3480

tctcctacag agcctcattg agtcccatta atgcttacga attcaatttt caggtgcgaa 3540

gcagagttca ttaaaaaaat tgttgatgag aatatttggg aatggctccc cagaacaaac 3600

gaattacatg tggccaggca cccaattgga atcaaatctc gcattcaagg tattatcagt 3660

gatctttcaa gtggtggatc aaatgatgtt ctcatggttg gaatttgggg gatgggtgga 3720

ttgggtaaaa caacagctgc caaagccatt tataaccaaa ttcatcatat gtttgaattc 3780

aaaagtttcc ttgccgacgt tagcgacact tcaagtaaac atggtctggt ttatttgcaa 3840

gaaacactta tttctgacat cttgaaacag aagtctcaaa taagaaatgt tgacttaggt 3900

atcaatatga tacaacaagt agttaaacat agaagggtac ttatcatcat tgacaatata 3960

gatgaccgga aacaactgga agcaatagcc caaagtcacg attggtttgg tccaggaagt 4020

agaattatca tgacgacacg gaatgaacat ttactaaaga aagtggaagt ggacaagaca 4080

tatccgcttc gggaaatgaa aaaggaagaa gctctggagc tctttagttg gcatgccttt 4140

caaaaaagtt gccctaatga agaatatctt gaagtctcaa aaaaggttgt ttcttattgt 4200

ggaggtttgc cactagccct tgaagtttta ggttctcttt tgcttaaaag acccattata 4260

gagtggaaaa gacaattgga gaaattagaa agaattcctg aaggagaaat aataaaacca 4320

ctaagaataa gctttgaagg gctagatgat acagagaagg ctacattcct tgacatatct 4380

tgtttcttta ttggaaagga aaaggactat gttgccaaaa tattagacgg atgtggattt 4440

tatgcaacat taggaatcag tatccttcgc gaacgatgtc ttgtaactgt tgaatacaat 4500

cgtttgaaaa tgcatgattt gattcgagaa atggccagag taatcatttc tgaaaaatct 4560

cttgatcgcc ctggaaaatg gagtaggctg tggaaccgtc aagaggtcac cgatgtattg 4620

acaaataaat ctgtaagttc attcccacta aattttggat ttatgcgtca tacaagaaaa 4680

gcatatatgt aacccgtgca agtcaagtat acataaacaa cattcatgtg taaatatatt 4740

taccgtacgt gattttgtgt aatgaataat aataataaat gtaaacctat gcaaatatac 4800

cgtttcttac ctctataact tttttttttt ttttgttaac agggaactgg aaaaattgaa 4860

ggacttgctc tagagtgccc tcctgatcct ttgttttcat tctttggttt cagtacaaaa 4920

gctgctagtt tcagtacaga agcatttgcc aatatgaaga aactgagatt gcttgagctc 4980

aactacgtag agctcaaagg agaatacaaa catcttccca aagagttaat atggttgtgt 5040

tgggaaggat tccctttaca gtccatacca gatgactttt ttaatcaaga taaactagtt 5100

gttttagaga tgcacgggag cgatctggta caagtttggg agggttccaa ggtacaatga 5160

attacacctg atgatgttat gtttctatat gttttttttt tttcataaca tcttcatttc 5220

ggatattaat ttcaatcttc ttttgatttt ctgcaacagt cgcttcgtaa cttgaaaacc 5280

cttgatctct gttgttcctc gtccttacag aaaccaccgg acttttcaca agtcccaaat 5340

cttgaagagc tgatattgga agggtgttct agtttgttcg agattcaccc ctctattggt 5400

tatcttaaaa gactttcttt ggtgaacctt aatggctgtt acagtctcag ttctcttcca 5460

agggatttct ataggtcgaa atctgttgag actcttcttc ttaatagatg tacaaaattc 5520

agagaactgc atgaggatgt aggggagatg atatcactga gaacacttga agcaaaggat 5580

acagccataa gagaagtacc acattccatt ggtcatctta aaagactttc tttggtgaac 5640

cttacccgat gttgggaact caggtctctt ccaagggatt tctataagtc gaaatctgtt 5700

gagactcttc ttcttaatag atgtacgaaa ttcatagaac tgcatgagga tataggggaa 5760

atgatatcac tgagaacact tgaagcaatg tttacagcca taagagaagt accaccttcc 5820

atagtaagat tgaagaatct cactcgttta tccctggact gcatcagcgt agagctcaag 5880

ggagaataca aatatctgtg ttggaaacga tgccctttaa agtccatacc aggtgacttt 5940

tttaatcaag atagactagt tgttttagag atgccgcaaa gcaaactggt acaagtttgg 6000

gagggttcca aggtacaatg aattacacct gatgatgttc tgtttctatt tgttttttgg 6060

attttttttt tttttataac atcttccttt cggatattaa tttcaatttt cttttgattt 6120

tctgcaacag tcgctacata acttgaaaac ccttgatctc agctattcct attccttaca 6180

gaaatcaccg gacttttcac aagtcccaaa tcttgaagag ttgatattgg aatggtgttc 6240

tagtttgtcc gagattcacc cctccattgg tcatcttaaa agactttctt tggtgaacct 6300

taatggctgt tacagtctca gttctcttcc aagggatttc tataagtcga aatctgttga 6360

gactcttctt cttaatagat gtacgaaatt tagagaactg cataaggata taggggagat 6420

gatatcactg agaacacttg aagcagagga tacagccata agagaagtac caccttccat 6480

agtaggattg aagaatctca ctcgtttatc aggtgtggga agtattaaac ttgaccgcat 6540

cagcgtaaag catacagcca aagagttaat acggttgcgt tggaaaggat gccctttaaa 6600

gtccatacca ggtgactttt ttaatcaaga tagactagtt gttttagaga tgccgcaaag 6660

caaactggta caagtttggg agggttccaa ggtacaatga attacacctg atgatgttct 6720

gtttctattt gttttttgga tttttttttt tttttataac atcttccttt cggatattaa 6780

tttcaatttt cttttgattt tctgcaacag tcgctacata acttgaaaac ccttgatctc 6840

agcggttcct ggtccttaca gaaatcacta gacttttcac aagtcccaaa tcttgaagag 6900

ttgatattgg aagggtgtga gagtttgttc gagattcacc cctcaattgg tcatcttaaa 6960

agactttctt tggtgaacct tggagaatgc gataagctta tttctcttcc aagggatttc 7020

tttaagtcga aatctgttga gactcttctt cttaattact gttcagaatt cattgaactg 7080

catgaggata taggggagat gaaatcactg agaacacttg aagcaatgtt tacagccata 7140

agagaagtac caccttccat tggtcatctt aaaagacttt ctttggtgaa cctcacaggc 7200

tgcgacaatc ttatttctct tccaagggat ttctataaat tgaaatctat tgagactctt 7260

cttcttaatg gatgtttaga attcatagaa ctgcatgagg atatatggga gatgatatca 7320

ctgagaacac ttgaagcaaa ggatacagcc ataaaagaag taccacattc catagtaaga 7380

ttgcagaatc tcactcgttt atacctggac aagtactgcc tgagcataga gctcaaagga 7440

gaatacaaat atctgtgttg ggacctatgc cctttaaagt tcataccaga tgactcgttt 7500

atcttggact tttttaatca agatcaacta gttcgtttaa agatgcatgg gagccaactg 7560

gtacaagttt gggagggttc taaggtacaa tgaattacac ctgatgatgt tctgtttcta 7620

tttgtttttt ggattttttc tcatttttgt ttataacatc ttgtgttata aaggaaagct 7680

tatttattga tatccccgat aagctacaaa tatgtacata tacatgagtc aaaataaaca 7740

aacaagatgg agccttcaca aaggttgctt aggagaagtc tcagcagtcg gtagagcccc 7800

agaaagagaa ggcatcggag ggggatcatt cggagcctca gtactggaca gaaccctaga 7860

aggaggaggc atcagaggtt gatcatttgg agcttcatta cgcggtacag ccccagaaga 7920

cgaaggcaat aaatgccttt ggaacaaacc cacaaatctc tgatgatcaa gtaaaacctg 7980

accatcagtt tccttcatct ggtcaagctt cctcttcatg tttgtagcat agtcatgtgc 8040

gagccggtgc aactgtttat tctcgtgctt gagccctcta atctcctgtt tgagactcat 8100

cacttcggcc gccaatgatt caacttggcg ggttcgagca aataggcgtt gggccatatt 8160

agacacagaa cctgcacact gaacactgag agccagcgaa tccttaacag ccaactcatc 8220

agatcgtttg gaaagtagtc tgttatcttt gggagtgaga aggttcctgg ccaccaccgc 8280

agcggtcata tcattcttca tcacggaatc cccaacggta agaggaccag taggggagac 8340

gaaggatggg cgccatatgt tgtctggaga aggcggggct gcctcttcaa caaggttcaa 8400

gtcaaaacga cggtcggagg ggccagacat tttcaaaggt gttgaagaga gaagaggtcg 8460

gacaaatcaa gatcttagaa gtgcaagatt ggagcttcta ctggtggaga ttca 8514

<210> 38
<211> 30
<212> DNA
<213> Artificial Sequence

<220>
<221> source
<222> 1..30
<223> /mol_type="unassigned DNA"
      /note="primer"
      /organism="Artificial Sequence"

<400> 38
ccttgcgcgc cttgccggag atgagagaga 30

<210> 39
<211> 30
<212> DNA
<213> Artificial Sequence

<220>
<221> source
<222> 1..30
<223> /mol_type="unassigned DNA"
      /note="primer"
      /organism="Artificial Sequence"

<400> 39

ccttacgcgt ctaaccttaa cactatgtgt                                        30


<210> 40
<211> 29
<212> DNA
<213> Artificial Sequence

<220>
<221> source
<222> 1..29
<223> /mol_type="unassigned DNA"
      /note="primer"
      /organism="Artificial Sequence"

<400> 40
ccttgcgcgc cattcctcca gcccaccaa                                         29


<210> 41
<211> 28
<212> DNA
<213> Artificial Sequence

<220>
<221> source
<222> 1..28
<223> /mol_type="unassigned DNA"
      /note="primer"
      /organism="Artificial Sequence"

<400> 41
ccttacgcgt gaatctccac cagtagaa                                          28


<210> 42
<211> 31
<212> DNA
<213> Artificial Sequence

<220>
<221> source
<222> 1..31
<223> /mol_type="unassigned DNA"
      /note="primer"
      /organism="Artificial Sequence"

<400> 42
ccttgcgcgc ctggttggtt cggtttaaga c                                      31


<210> 43
<211> 39
<212> DNA
<213> Artificial Sequence

<220>
<221> source
<222> 1..39
<223> /mol_type="unassigned DNA"
      /note="primer"
      /organism="Artificial Sequence"

<400> 43
ccttgctagc tgcgcgcgaa taagtggcaa tggtagaac                              39

<210> 44
<211> 20
<212> DNA
<213> Artificial Sequence

<220>
<221> source
<222> 1..20
<223> /mol_type="unassigned DNA"
      /note="primer"
      /organism="Artificial Sequence"

<400> 44
ttcgataatg gaggcaagtc                                                   20


<210> 45
<211> 20
<212> DNA
<213> Artificial Sequence

<220>
<221> source
<222> 1..20
<223> /mol_type="unassigned DNA"
      /note="primer"
      /organism="Artificial Sequence"

<400> 45
caagttttg cggtttggtt                                                    20


<210> 46
<211> 20
<212> DNA
<213> Artificial Sequence

<220>
<221> source
<222> 1..20
<223> /mol_type="unassigned DNA"
      /note="primer"
      /organism="Artificial Sequence"

<400> 46
taggcgttgg gccatattag                                                   20


<210> 47
<211> 21
<212> DNA
<213> Artificial Sequence

<220>
<221> source
<222> 1..21
<223> /mol_type="unassigned DNA"
      /note="primer"
      /organism="Artificial Sequence"

<400> 47
gcaattagct gtgcaaacca t                                                 21

<210> 48
<211> 22
<212> DNA
<213> Artificial Sequence

<220>
<221> source
<222> 1..22
<223> /mol_type="unassigned DNA"
      /note="primer"
      /organism="Artificial Sequence"

<400> 48
catttgcaag actcagaaat ga                                              22


<210> 49
<211> 22
<212> DNA
<213> Artificial Sequence

<220>
<221> source
<222> 1..22
<223> /mol_type="unassigned DNA"
      /note="primer"
      /organism="Artificial Sequence"

<400> 49
tcgaaatgtt ttcattcctt cc                                              22


<210> 50
<211> 22
<212> DNA
<213> Artificial Sequence

<220>
<221> source
<222> 1..22
<223> /mol_type="unassigned DNA"
      /note="primer"
      /organism="Artificial Sequence"

<400> 50
ctgcaaggcg attaagttgg gt                                              22


<210> 51
<211> 20
<212> DNA
<213> Artificial Sequence

<220>
<221> source
<222> 1..20
<223> /mol_type="unassigned DNA"
      /note="primer"
      /organism="Artificial Sequence"

<400> 51
cttacctact gcttccagac                                                 20

<210> 52
<211> 1095
<212> PRT
<213> Malus sp.

<220>
<223> [CDS]:1..3288 from SEQ ID NO 3

<400> 52
Met Thr Ala His Glu Ala Ser Ser Ser Ser Ser Ser Lys Ser Lys Leu
1               5                   10                  15
Trp Ser Tyr Asp Val Phe Leu Ser Phe Arg Gly Glu Asp Thr Arg Asn
            20                  25                  30
Gly Phe Thr Ser His Leu His Glu Ala Leu Lys Tyr Arg Gly Tyr His
        35                  40                  45
Val Phe Ile Asp Glu Asp Gly Leu Lys Arg Gly Glu Glu Ile Lys Glu
    50                  55                  60
Lys Leu Phe Arg Ala Ile Glu Glu Ser Arg Ile Ser Ile Ile Val Phe
65                  70                  75                  80
Ser Lys Met Tyr Ala Asp Ser Ser Trp Cys Leu Asp Glu Leu Val Lys
                85                  90                  95
Ile Met Glu Cys Arg Asp Lys Leu Gly Gln His Val Leu Pro Ile Phe
            100                 105                 110
Tyr His Val Asp Pro Ser His Val Arg Lys Gln Asp Gly Asp Leu Ala
        115                 120                 125
Glu Ala Phe Gln Lys His Glu Lys Asp Ile Arg Glu Glu Lys Asp Asp
    130                 135                 140
Glu Lys Arg Glu Ala Lys Gln Lys Arg Ala Glu Gln Trp Lys Gly Ala
145                 150                 155                 160
Leu Thr Lys Ala Ala Asp Leu Ser Gly His His Leu Lys Ser Thr His
                165                 170                 175
Asn Arg Glu Arg Arg Arg Asp Pro Leu Thr Glu Ala Ala Asn Leu Ser
            180                 185                 190
Asp His Gln Thr Thr Asp Asn Gly Ser Arg Lys Gly Lys Thr Phe Pro
        195                 200                 205
Leu Arg Ile Thr Thr Phe Asn Arg Cys Glu Ala Lys Phe Ile Lys Lys
    210                 215                 220
Ile Val Asp Glu Asn Ile Trp Glu Trp Leu Pro Arg Thr Asn Glu Leu
225                 230                 235                 240
His Val Ala Lys His Pro Ile Gly Ile Lys Ser Arg Ile Gln Gly Ile
                245                 250                 255
Ile Ser Asp Leu Ser Ser Gly Gly Ser Asn Asp Val Leu Met Val Gly
            260                 265                 270
Ile Trp Gly Met Gly Gly Leu Gly Lys Thr Thr Val Ala Lys Ala Ile
        275                 280                 285
Tyr Asn Gln Ile His Arg Lys Phe Glu Phe Lys Ser Phe Leu Ala Asp
    290                 295                 300
Val Ser Asp Ala Thr Ser Lys His Gly Leu Val Tyr Leu Gln Glu Lys
305                 310                 315                 320
Leu Ile Ser Asp Ile Leu Lys Gln Lys Ser Gly Ser Ser Asn Phe Asp
                325                 330                 335
Glu Gly Ile Asn Leu Ile Lys Gln His Leu Gln Arg Arg Arg Val Leu
            340                 345                 350
Val Ile Ile Asp Asn Ile Asp Asp Leu Lys Gln Leu Asp Ala Ile Ala
        355                 360                 365
Val Ser His His Cys Phe Gly Pro Gly Ser Arg Ile Ile Ile Thr Thr
    370                 375                 380
Arg Asn Glu His Leu Leu Lys Gln Val Glu Val Asp Lys Thr Tyr Pro
385                 390                 395                 400
Leu Gln Glu Met Lys Lys Lys Glu Ala Leu Glu Leu Phe Ser Trp His
                405                 410                 415
Ala Phe Arg Lys Ser Tyr Pro Asn Glu Glu Tyr Leu Glu Val Ser Glu
            420                 425                 430
Lys Val Val Ser Tyr Cys Gly Gly Leu Pro Leu Ala Leu Glu Val Leu
        435                 440                 445

Gly Ser Phe Leu Phe Lys Arg Pro Ile Ala Glu Trp Lys Ser Gln Leu
    450                 455                 460
Lys Lys Leu Glu Arg Thr Pro Asn Gly Glu Ile Ile Lys Pro Leu Lys
465                 470                 475                 480
Ile Ser Phe Glu Gly Leu Asp Asp Thr Glu Lys Ala Ile Phe Leu Asp
                485                 490                 495
Ile Ser Cys Phe Phe Ile Gly Arg Asp Lys Asp Tyr Val Ala Lys Val
            500                 505                 510
Leu Asp Gly Cys Gly Phe Phe Ala Thr Gly Gly Ile Ser Val Leu Arg
        515                 520                 525
Glu Arg Cys Leu Val Thr Val Glu Gly Asn Lys Leu Thr Met His Asp
    530                 535                 540
Leu Leu Arg Glu Met Ala Arg Val Ile Ile Ser Glu Lys Ser Leu Asp
545                 550                 555                 560
Cys Pro Gly Lys Trp Ser Arg Leu Trp Asn Arg Gln Glu Val Thr Asp
                565                 570                 575
Val Leu Thr Asn Lys Ser Gly Thr Gly Glu Ile Glu Gly Leu Ala Leu
            580                 585                 590
Tyr Phe Pro Lys Glu Thr Lys Ile Ala Ser Phe Ser Thr Lys Ala Phe
        595                 600                 605
Ala Ile Met Lys Lys Leu Ser Leu Leu Ala Leu Leu Phe Arg Lys Glu
    610                 615                 620
Thr Lys Ile Ala Ser Phe Ser Thr Lys Ala Phe Ala Lys Met Lys Lys
625                 630                 635                 640
Leu Arg Leu Leu Gln Leu Ser Asn Val Glu Leu Lys Gly Glu Tyr Lys
                645                 650                 655
His Leu Pro Lys Glu Leu Ile Trp Leu Arg Trp Glu Gly Cys Pro Leu
            660                 665                 670
Lys Ser Leu Pro Asp Asp Phe Phe Asn Gln Pro Arg Leu Val Val Leu
        675                 680                 685
Glu Met Gln Cys Ser Lys Leu Val Gln Val Trp Lys Gly Phe Lys Ser
    690                 695                 700
Leu His Asn Leu Lys Thr Leu Asp Leu Ser Trp Ser Asp Ser Leu Gln
705                 710                 715                 720
Lys Ser Pro Asp Phe Ser Gln Val Pro Asn Leu Glu Glu Leu Ile Leu
                725                 730                 735
Glu Ala Cys Ile Asn Leu Ser Glu Ile His Pro Ser Ile Gly His Leu
            740                 745                 750
Lys Arg Leu Ser Leu Val Asn Leu Lys Trp Cys Asn Lys Leu Ile Ser
        755                 760                 765
Leu Pro Arg Asp Phe Tyr Lys Leu Lys Ser Val Glu Thr Leu Leu Leu
    770                 775                 780
Asn Asn Ser Tyr Gln Phe Arg Glu Val His Glu Asp Ile Gly Glu Met
785                 790                 795                 800
Ile Ser Leu Arg Thr Leu Glu Ala Arg Gly Thr Ala Ile Arg Glu Val
                805                 810                 815
Pro Pro Ser Ile Val Gly Leu Lys Asn Leu Thr Arg Leu Ser Leu Glu
            820                 825                 830
Gly Thr Met Arg Gly Ile Gln Leu Pro His Ser Leu Arg Glu Ile Asp
        835                 840                 845
Leu Thr Tyr Gly Ala Leu Asp Asp Ala Ile Lys Asp Leu Gly Ser Leu
    850                 855                 860
Ile Tyr Leu Gln His Leu His Leu Gln Gly Asn Asn Phe His Ser Leu
865                 870                 875                 880
Pro Asn Leu Ser Gly Leu Leu Lys Leu Glu Thr Leu Trp Leu Asn Gly
                885                 890                 895
Ser Glu Tyr Leu Arg Thr Ile Pro Asp Leu Pro Thr Asn Leu Lys Val
            900                 905                 910
Leu Phe Ala Asp Ala Cys Pro Ala Leu Glu Thr Met Pro Asp Phe Ser
        915                 920                 925
Glu Met Ser Asn Met Arg Thr Leu Asp Val Ser His Ser Ala Lys Leu
    930                 935                 940
Thr Gly Val Pro Gly Leu Asp Lys Ser Leu Asn Ser Met Val Trp Leu
945                 950                 955                 960
Asp Met His Arg Cys Pro Asn Leu Thr Ala Asp Phe Arg Lys Asn Ile

965                 970                 975
Leu Gln Gly Trp Thr Ser Cys Gly Phe Gly Gly Ile Ala Leu His Gly
            980                 985                 990
Asn Tyr Val Pro Asp Trp Phe Glu Phe Ile Asn Glu Gly Thr Lys Arg
        995                 1000                1005
Thr Lys Leu Arg Ala Tyr Lys Thr Lys Ala Ser Tyr Thr Ile Asp Glu
    1010                1015                1020
Asn Tyr Glu Asn Asp His Leu Trp Gln Gly Gln Phe Ser Asn Asn Glu
1025                1030                1035                1040
Leu Asn Leu Gln Gly Gly Asp Lys Val Asp Ile Val Phe Glu Asn Leu
                1045                1050                1055
Asp Glu Asp Asp Ser Val Lys Met Met Arg Thr Gly Val Asn Leu Val
            1060                1065                1070
Trp Asp Lys Leu Met Lys Glu Asn Met His Asp Leu Gly Asp Thr Arg
        1075                1080                1085
Gly Arg Ile Ser Phe Gly Ile
    1090                1095

<210> 53
<211> 1095
<212> PRT
<213> Malus sp.

<220>
<223> [CDS]:join(3963..4496,4597..4661,5976..6025,6224..7321,7533..7894
      ,8016..8841,8933..9018,9145..9411) from SEQ ID NO 2

<400> 53
Met Thr Ala His Glu Ala Ser Ser Ser Ser Ser Ser Lys Ser Lys Leu
1               5                   10                  15
Trp Ser Tyr Asp Val Phe Leu Ser Phe Arg Gly Glu Asp Thr Arg Asn
            20                  25                  30
Gly Phe Thr Ser His Leu His Glu Ala Leu Lys Tyr Arg Gly Tyr His
        35                  40                  45
Val Phe Ile Asp Glu Asp Gly Leu Lys Arg Gly Glu Glu Ile Lys Glu
    50                  55                  60
Lys Leu Phe Arg Ala Ile Glu Glu Ser Arg Ile Ser Ile Ile Val Phe
65                  70                  75                  80
Ser Lys Met Tyr Ala Asp Ser Ser Trp Cys Leu Asp Glu Leu Val Lys
                85                  90                  95
Ile Met Glu Cys Arg Asp Lys Leu Gly Gln His Val Leu Pro Ile Phe
            100                 105                 110
Tyr His Val Asp Pro Ser His Val Arg Lys Gln Asp Gly Asp Leu Ala
        115                 120                 125
Glu Ala Phe Gln Lys His Glu Lys Asp Ile Arg Glu Glu Lys Asp Asp
    130                 135                 140
Glu Lys Arg Glu Ala Lys Gln Lys Arg Ala Glu Gln Trp Lys Gly Ala
145                 150                 155                 160
Leu Thr Lys Ala Ala Asp Leu Ser Gly His His Leu Lys Ser Thr His
                165                 170                 175
Asn Arg Glu Arg Arg Arg Asp Pro Leu Thr Glu Ala Ala Asn Leu Ser
            180                 185                 190
Asp His Gln Thr Thr Asp Asn Gly Ser Arg Lys Gly Lys Thr Phe Pro
        195                 200                 205
Leu Arg Ile Thr Thr Phe Asn Arg Cys Glu Ala Lys Phe Ile Lys Lys
    210                 215                 220
Ile Val Asp Glu Asn Ile Trp Glu Trp Leu Pro Arg Thr Asn Glu Leu
225                 230                 235                 240
His Val Ala Lys His Pro Ile Gly Ile Lys Ser Arg Ile Gln Gly Ile
                245                 250                 255
Ile Ser Asp Leu Ser Ser Gly Gly Ser Asn Asp Val Leu Met Val Gly
            260                 265                 270
Ile Trp Gly Met Gly Gly Leu Gly Lys Thr Thr Val Ala Lys Ala Ile
        275                 280                 285
Tyr Asn Gln Ile His Arg Lys Phe Glu Phe Lys Ser Phe Leu Ala Asp

290                 295                 300
Val Ser Asp Ala Thr Ser Lys His Gly Leu Val Tyr Leu Gln Glu Lys
305                 310                 315                 320
Leu Ile Ser Asp Ile Leu Lys Gln Lys Ser Gly Ser Ser Asn Phe Asp
                325                 330                 335
Glu Gly Ile Asn Leu Ile Lys Gln His Leu Gln Arg Arg Arg Val Leu
            340                 345                 350
Val Ile Ile Asp Asn Ile Asp Asp Leu Lys Gln Leu Asp Ala Ile Ala
        355                 360                 365
Val Ser His His Cys Phe Gly Pro Gly Ser Arg Ile Ile Ile Thr Thr
    370                 375                 380
Arg Asn Glu His Leu Leu Lys Gln Val Glu Val Asp Lys Thr Tyr Pro
385                 390                 395                 400
Leu Gln Glu Met Lys Lys Lys Glu Ala Leu Glu Leu Phe Ser Trp His
                405                 410                 415
Ala Phe Arg Lys Ser Tyr Pro Asn Glu Glu Tyr Leu Glu Val Ser Glu
            420                 425                 430
Lys Val Val Ser Tyr Cys Gly Gly Leu Pro Leu Ala Leu Glu Val Leu
        435                 440                 445
Gly Ser Phe Leu Phe Lys Arg Pro Ile Ala Glu Trp Lys Ser Gln Leu
    450                 455                 460
Lys Lys Leu Glu Arg Thr Pro Asn Gly Glu Ile Ile Lys Pro Leu Lys
465                 470                 475                 480
Ile Ser Phe Glu Gly Leu Asp Asp Thr Glu Lys Ala Ile Phe Leu Asp
                485                 490                 495
Ile Ser Cys Phe Phe Ile Gly Arg Asp Lys Asp Tyr Val Ala Lys Val
            500                 505                 510
Leu Asp Gly Cys Gly Phe Phe Ala Thr Gly Gly Ile Ser Val Leu Arg
        515                 520                 525
Glu Arg Cys Leu Val Thr Val Glu Gly Asn Lys Leu Thr Met His Asp
    530                 535                 540
Leu Leu Arg Glu Met Ala Arg Val Ile Ile Ser Glu Lys Ser Leu Asp
545                 550                 555                 560
Cys Pro Gly Lys Trp Ser Arg Leu Trp Asn Arg Gln Glu Val Thr Asp
                565                 570                 575
Val Leu Thr Asn Lys Ser Gly Thr Gly Glu Ile Glu Gly Leu Ala Leu
            580                 585                 590
Tyr Phe Pro Lys Glu Thr Lys Ile Ala Ser Phe Ser Thr Lys Ala Phe
        595                 600                 605
Ala Ile Met Lys Lys Leu Ser Leu Leu Ala Leu Leu Phe Arg Lys Glu
    610                 615                 620
Thr Lys Ile Ala Ser Phe Ser Thr Lys Ala Phe Ala Lys Met Lys Lys
625                 630                 635                 640
Leu Arg Leu Leu Gln Leu Ser Asn Val Glu Leu Lys Gly Glu Tyr Lys
                645                 650                 655
His Leu Pro Lys Glu Leu Ile Trp Leu Arg Trp Glu Gly Cys Pro Leu
            660                 665                 670
Lys Ser Leu Pro Asp Asp Phe Phe Asn Gln Pro Arg Leu Val Val Leu
        675                 680                 685
Glu Met Gln Cys Ser Lys Leu Val Gln Val Trp Lys Gly Phe Lys Ser
    690                 695                 700
Leu His Asn Leu Lys Thr Leu Asp Leu Ser Trp Ser Asp Ser Leu Gln
705                 710                 715                 720
Lys Ser Pro Asp Phe Ser Gln Val Pro Asn Leu Glu Glu Leu Ile Leu
                725                 730                 735
Glu Ala Cys Ile Asn Leu Ser Glu Ile His Pro Ser Ile Gly His Leu
            740                 745                 750
Lys Arg Leu Ser Leu Val Asn Leu Lys Trp Cys Asn Lys Leu Ile Ser
        755                 760                 765
Leu Pro Arg Asp Phe Tyr Lys Leu Lys Ser Val Glu Thr Leu Leu Leu
    770                 775                 780
Asn Asn Ser Tyr Gln Phe Arg Glu Val His Glu Asp Ile Gly Glu Met
785                 790                 795                 800
Ile Ser Leu Arg Thr Leu Glu Ala Arg Gly Thr Ala Ile Arg Glu Val
                805                 810                 815

Pro Pro Ser Ile Val Gly Leu Lys Asn Leu Thr Arg Leu Ser Leu Glu
            820                 825                 830
Gly Thr Met Arg Gly Ile Gln Leu Pro His Ser Leu Arg Glu Ile Asp
        835                 840                 845
Leu Thr Tyr Gly Ala Leu Asp Asp Ala Ile Lys Asp Leu Gly Ser Leu
    850                 855                 860
Ile Tyr Leu Gln His Leu His Leu Gln Gly Asn Asn Phe His Ser Leu
865                 870                 875                 880
Pro Asn Leu Ser Gly Leu Leu Lys Leu Glu Thr Leu Trp Leu Asn Gly
                885                 890                 895
Ser Glu Tyr Leu Arg Thr Ile Pro Asp Leu Pro Thr Asn Leu Lys Val
            900                 905                 910
Leu Phe Ala Asp Ala Cys Pro Ala Leu Glu Thr Met Pro Asp Phe Ser
        915                 920                 925
Glu Met Ser Asn Met Arg Thr Leu Asp Val Ser His Ser Ala Lys Leu
    930                 935                 940
Thr Gly Val Pro Gly Leu Asp Lys Ser Leu Asn Ser Met Val Trp Leu
945                 950                 955                 960
Asp Met His Arg Cys Pro Asn Leu Thr Ala Asp Phe Arg Lys Asn Ile
                965                 970                 975
Leu Gln Gly Trp Thr Ser Cys Gly Phe Gly Gly Ile Ala Leu His Gly
            980                 985                 990
Asn Tyr Val Pro Asp Trp Phe Glu Phe Ile Asn Glu Gly Thr Lys Arg
        995                 1000                1005
Thr Lys Leu Arg Ala Tyr Lys Thr Lys Ala Ser Tyr Thr Ile Asp Glu
    1010                1015                1020
Asn Tyr Glu Asn Asp His Leu Trp Gln Gly Gln Phe Ser Asn Asn Glu
1025                1030                1035                1040
Leu Asn Leu Gln Gly Gly Asp Lys Val Asp Ile Val Phe Glu Asn Leu
                1045                1050                1055
Asp Glu Asp Asp Ser Val Lys Met Met Arg Thr Gly Val Asn Leu Val
            1060                1065                1070
Trp Asp Lys Leu Met Lys Glu Asn Met His Asp Leu Gly Asp Thr Arg
        1075                1080                1085
Gly Arg Ile Ser Phe Gly Ile
    1090                1095

## Description

This invention relates to a method for increasing resistance against a pathogen in a perennial plant, in particular an apple plant (Malus). The invention also relates to an isolated polynucleotide for use in this method and a perennial plant with increased resistance against a pathogen or parasite compared to its corresponding wild type.

### Background of the invention

Fruit pathogens cause serious losses to the fruit industry because of diseases. One of the major diseases that lead to lowering of fruit quality is scab. Scab can defoliate a tree. In addition, the fruit can become malformed, cracked, and unsightly, which renders it unsaleable. To some of the major fruit crops scab is a major problem.

For example, apple is one of the major fruit crops in temperate regions. Apple scab, caused by the fungus *Venturia inaequalis,* is a major disease of apple, especially in humid climates. Also in pear, scab is a major problem. Pear scab, which is caused by the fungus *Venturia pirina,* results in comparable blemishes on pear fruit as in apples.

In order to overcome the problem of apple scab disease, already in 1914 crosses were made between a susceptible cultivar and the resistant crab apple *Malus floribunda* 821 (Crandall 1926; Hough et al. 1953; Gessler and Pertot, 2011). The progeny of this cross segregated in a Mendelian fashion for resistance in a 1:1 ratio. The gene putatively underlying this resistance was named *Vf*-gene. However, the fruits of the resistant parent *M*. *floribunda* 821 were very small, approximately 1 cm. The apples of the progeny were small too, and did not have the fruit quality that was required for commercial cultivars. This was caused by linkage drag: not only the wanted resistance gene was inherited to part of the progeny, but also many unwanted alleles, leading to poor fruit quality and other unwanted traits (Schouten et al, 2009). In order to get rid of the unwanted alleles, subsequent crosses had to be carried out between resistant progeny and susceptible high quality cultivars. About five generations were required to remove the far majority of the unwanted alleles from *M*. *floribunda,* yet keeping the desired *Vf*-gene for scab resistance. Approximately 80 years after the first cross, Vf-cultivars with a reasonable fruit quality were introduced onto the market (Anonymous, 1999). It took more than half a century to introduce the *Vf*-gene and remove the linkage drag to a reasonable degree.

In the mean time, *V*. *inaequalis* strains were detected that are able to infect Vf-cultivars (Parisi et al., 1993). Especially in northwestern Europe, these strains are present and have spread (Parisi et al., 2006). As a result, several orchards that consist of Vf-cultivars have to be treated with fungicide like orchards with susceptible cultivars (Trapman, 2006). It is therefore clear that in order to obtain more durable resistance towards apple scab, more individual resistance genes need to be accumulated in apple cultivars.

Several loci that confer resistance to apple scab have been discovered in *Malus,* both major genes and quantitative trait loci (QTLs) (Calenge et al., 2004; Gardiner et al., 2006; Gessler et al., 2006). Therefore, sufficient genes for resistance are present in the germplasm of apple. Introgression of one resistance gene took approximately 80 years. Introgression of two or more genes for durable resistance will require more time, when the breeding is performed in the classical way.

One way to speedup the breeding process is marker-assisted breeding. Accumulation of resistance genes from several sources of resistance and sufficient removal of unwanted alleles from these sources, will probably require at least another four generations of apple. As long as the juvenile period and additional evaluation time in apple is about eight years, this would still require a minimum of 32 years of breeding.

The abovementioned problems are also relevant to fruit plants other than apple plants.

To overcome these problems the present invention aims to make resistance towards pathogens in perennial plants more durable and to shorten the time needed to produce a pathogen resistant perennial plant.

### Summary of the invention

This invention relates to a method for increasing resistance against a pathogen in a perennial plant, comprising incorporating into the genome of the perennial plant a gene encoding a protein having the sequence as set forth in SEQ ID NO: 1 or a sequence with at least 70% sequence identity thereto. Said gene is preferably derived from a plant that is sexually compatible with said perennial plant. Accordingly the invention also relates to a plant obtained by the method of the invention.

In another aspect the invention relates to an isolated nucleic acid molecule. In particular the invention relates to an isolated nucleic acid molecule derived from an apple scab resistant apple plant comprising a nucleotide sequence that encodes a protein having the sequence as set forth in SEQ ID NO: 1 or a sequence with at least 70% sequence identity thereto. The abovementioned isolated nucleic acid molecule may comprise or have the sequence as set forth in SEQ ID NO: 3 or a sequence with at least 90% sequence identity to SEQ ID NO: 3; or may be a polynucleotide hybridising selectively under stringent conditions thereto, or a sequence complementary thereto. Alternatively, the abovementioned isolated nucleic acid molecule may comprise or have the sequence as set forth in SEQ ID NO: 2 or a sequence wherein the nucleic acid sequences at the positions which correspond, as determined by sequence alignment, to the nucleic acid sequences that are represented by residues 3963-4496, 4597-4661, 5976-6025, 6224-7321, 7533-7894, 8016-8841, 8933-9018 and 9145-9411 of SEQ ID NO: 2 are at least 90 % identical; or may be a polynucleotide hybridising selectively under stringent conditions thereto, or a sequence complementary thereto.

The invention thus allows introduction of a resistance gene into elite cultivars without simultaneous introgression of unwanted alleles, so prevention of linkage drag, rather than removal of linkage drag. Then, resistance provided by a resistance gene is added to high quality cultivars in one step, preserving the proven fruit quality and other desired traits of these cultivars, thereby saving a considerable amount of time.

### Short description of the figures

Figure 1. The observed sporulation of the transformation events, containing candidate genes *Vr2-A, Vr2-B* or *Vr2-C.*
Figure 2. Disease symptoms on leaves of the apple cultivar 'Gala' after inoculation with *Venturia inaequalis.*

### Detailed description

The inventor has found that introducing a gene encoding a protein with a Toll and mammalian interleukin-1 receptor protein nucleotide-binding site leucine-rich repeat (TIR NBS LRR) structure into the genome of a perennial plant leads to a plant with increased resistance towards pathogens.

The gene introduced into the genome of the perennial plant is preferably derived from a plant that is sexually compatible with said fruit plant. If a natural gene from the same species or a gene from a plant that is sexually compatible with a host plant by means of conventional breeding methods is used to obtain an organism, said organism is called cisgenic, and the process by which this is achieved is named cisgenesis. A prerequisite of cisgenesis is the availability of isolated functional alleles. The already mentioned *Vf*-gene was isolated by means of map-based cloning and subsequently functionally analyzed (Belfanti et al., 2004; Joshi et al., 2011). Belfanti et al. (2004) named the gene *HcrVf2.* In the present invention a gene encoding a protein with a TIR NBS LRR structure was isolated from *Malus sp.* and used for cisgenesis. The gene of the invention is therefore preferably derived from a pathogen resistant cultivar or wild plant of the corresponding genus.

In addition to incorporating a gene encoding a protein having the sequence as set forth in SEQ ID NO: 1 or a sequence with at least 70% sequence identity thereto into the genome of a perennial plant, in the method of the invention also at least one further pathogen resistance gene may be incorporated into the genome of the perennial plant. Such pathogen resistance gene may be the abovementioned *HcrVf2*, one or more genes conferring resistance against fire blight caused by the bacterium *Erwinia amylovora,* or one or more genes conferring resistance against mildew (*Podosphaera leucotricha*).

The perennial plant of the invention in which pathogen resistance is increased may be any plant that is susceptible of developing scab, such as apple (Malus), pear (Pyrus) or peach (Prunus). Preferably this perennial plant is an apple cultivar (Malus x domesticus).

The invention also makes it possible to introduce an additional resistance gene into plant that already have one or more resistance genes. This will broaden the spectrum of diseases against which such a plant is resistant. In case the plant contains already one or more resistance genes to scab, incorporation of the abovementioned gene can improve the durability of the resistance.

In case the perennial plant is an apple plant, the gene encoding the protein with a TIR NBS LRR structure is preferably derived from an apple scab resistant apple (Malus sp.) plant, for instance from apple scab resistant plant GMAL 2473 (PI 589835, from the Geneva National Germplasm Repository).

The term "derived from" is meant to include a gene that originates from a selected fruit plant species as well as an artificial variant thereof. It is also possible that a gene originating from a selected perennial plant species, such as for instance apple scab resistant plant or genotype GMAL 2473 is artificially modified, but that this gene is applicable in the method of the invention. Such modifications may involve mutations and/or removal or replacement of intron sequences. The gene used in the method of the invention may be isolated from genomic DNA of a plant but may also be isolated from cDNA or RNA.

A pathogen, as referred to herein should be understood to be any microbe, such as a bacterium, fungus, nematode, insect or virus, that is able to infect a perennial plant leading to disease and economic damage. In the present invention the term "pathogen" may in particular refer to a pathogen that causes scab. Scab may be caused by various organisms such as fungi from the genus *Venturis*. Such a pathogen may for instance be *Venturia inaequalis,* which causes apple scab, *Venturia pirina,* which causes pear scab or *Venturia carpophila,* which causes peach scab.

Introducing the gene into a host perennial plant preferably involves *Agrobacterium tumefaciens* mediated transformation. A. *tumefaciens* mediated transformation is a relatively efficient means for transformation of many plant species and intact, unmodified genes are frequently integrated successfully into a plant's genome.

The gene used in the method of the invention may be the *Vr2* gene, which is identified herein by the inventors and which has been assigned with the name *Rvi15.* It appeared to be difficult to identify this gene, because in the plant's genome the gene contains introns and it is stretched out over a long range of DNA. This leads to a high risk of mistakes in PCR amplifications. Furthermore the members of the TIR NBS LRR gene family show high resemblance, which makes it even more difficult to amplify the correct gene from a plant's genomic DNA. The coding sequence of this gene is contained in the sequence of SEQ ID NO: 2. This sequence contains a promoter region (predicted to be comprised in the nucleotide sequence ranging from positions 1-3962 of SEQ ID NO:2), a terminator region (predicted to be comprised in the nucleotide sequence ranging from positions 9412-10282 of SEQ ID NO:2) and introns (predicted to be comprised in the nucleotide sequence ranging from positions 4497-4596, 4662-5975, 6026-6223, 7322-7532, 7895-8015, 8842-8932, 9019-9144 of SEQ ID NO: 2), which are removed by RNA splicing while the final mature RNA product of the gene is being generated based on the coding sequence. The gene (including introns) thus is predicted to range from positions 3963-9411 of SEQ ID NO: 2. The nucleotide sequences (exons) of which the transcript remains present within the final mature RNA product of that gene after introns have been removed by RNA splicing (i.e. the coding sequences) are predicted to be represented by the sequences ranging from 3963-4496, 4597-4661, 5976-6025, 6224-7321, 7533-7894, 8016-8841, 8933-9018 and 9145-9411 of SEQ ID NO: 2. The predicted coding sequence of the gene used in the method of the invention is set forth in SEQ ID NO: 3. The coding sequence as set forth in SEQ ID NO: 3 is predicted to encode a protein with the sequence as set forth in SEQ ID NO: 1. In the method of the invention a gene with introns or a gene without introns may be introduced into the genome of the perennial plant, preferably together with promoter and or terminator sequences operably linked to the gene, for instance the native promoter and terminator sequences of the gene. It should be understood that also genes homologous to the genes as shown in SEQ ID NO: 2 or 3 may be applicable in the method of the invention, provided that the coding sequence encodes a protein that has the same function (i.e. providing enhanced resistance) as the protein with the amino acid sequence as set forth in SEQ ID NO: 1 or a protein homologous thereto.

It will be understood that also functional derivatives of the TIR NBS TRR protein having the amino acid sequence of SEQ ID NO: 1 may be applicable in the method of the invention. The protein referred to in the method of the invention therefore also encompasses derivatives and homologues of SEQ ID NO: 1. The protein thus may have the sequence as set forth in SEQ ID NO: 1 or a sequence with at least 70% sequence identity thereto. More preferably such protein is a protein with between 75% and 100%, more preferably between 80% and 100%, more preferably between 85% and 100%, more preferably between 90% and 100%, more preferably between 95% and 100% sequence identity to SEQ ID NO: 1. The gene encoding this protein preferably comprises a nucleotide sequence as set forth in SEQ ID NO: 3 or a sequence with at least 90% sequence identity to SEQ ID NO: 3; or a nucleotide sequence as set forth in positions 3963-9411 of SEQ ID NO: 2 or a nucleotide sequence wherein the nucleic acid sequences at the positions which correspond, as determined by sequence alignment, to the nucleic acid sequences that are represented by residues 3963-4496, 4597-4661, 5976-6025, 6224-7321, 7533-7894, 8016-8841, 8933-9018 and 9145-9411 of SEQ ID NO: 2 are at least 90 % identical.

Polynucleotide sequences of the invention may be altered by substitutions, additions or deletions, i.e. silent mutations may be present. Silent mutations are DNA mutations that do not result in a change to the amino acid sequence of the encoded protein, because different codons may encode the same amino acid. Silent mutations may also result in amino acid change that does not result in substantially different properties of the changed amino acids. Silent mutations may occur or be introduced in a non-coding region, for instance outside of a gene, such as in a promoter or terminator region or within an intron, or they may occur within an exon in a manner that does not alter the behavior of the encoded protein. For example, several of the amino acids share similar properties. Arginine, lysine and histidine for example are positively charged at physiological pH. Aspartate and glutamate are negatively charged. Alanine, valine, isoleucine and leucine are small hydrophobic amino acids. Substitution of one or more amino acids within a protein sequence for another amino acid with the same properties therefore often leads to a functionally equivalent protein. For example, a substitution of valine for alanine within a protein sequence is usually not expected to influence protein functionality.

As mentioned above, the protein encoded by the gene of the invention comprises a) a Toll and mammalian interleukin-1 receptor protein (TIR) domain, b) a nucleotide binding site (NBS) domain, and c) a leucine-rich repeat (LRR) domain.

The N-terminal TIR domain may for instance be 232 amino acids long as set forth in SEQ ID NO: 4, containing the conserved regions SEQ ID NO: 5-9.

The NBS domain may for instance be 312 amino acids long as set forth in SEQ ID NO: 10, containing the conserved regions SEQ ID NO: 11-18.

The LRR domain may for instance be characterized by 15 regions with LRR motifs which have the amino acid sequence as set forth in SEQ ID NO: 19-33.

The gene of the invention may encode a TIR NBS LRR protein that contains a connecting domain between the NBS domain and the LRR domain represented by the sequence as set forth in SEQ ID NO: 34 and a C-terminal domain as set forth in SEQ ID NO: 35.

At some stage of the method of the invention the gene used in the method of the invention exists in the form of an isolated nucleic acid molecule. By "isolated" is meant material that is substantially or essentially free from components that normally accompany it in its native state. An example of such a molecule is an isolated nucleic acid molecule, which has been purified from the sequences which flank it in a naturally occurring state, e.g. a DNA fragment which has been removed from the sequences which are normally adjacent to the fragment. An isolated nucleic acid molecule may be obtained by genomic DNA isolation, digestion by endonuclease enzymes, PCR, DNA extraction, DNA purification or any method that will be known by the person skilled in the art. Isolated nucleic acid molecules may be obtained from any nucleic acid source, especially from biological samples or tissue samples. The isolated nucleic acid sequence may be derived from genomic DNA or directly from biological samples from a perennial plant. The term "biological samples" in the context of the invention is defined to include any sample derived from any part of a perennial plant.

The term "nucleic acid molecule" of the present invention encompasses derivatives, including mutants and homologues thereto. The term "nucleotide sequence" or "sequence of nucleotides" of the present invention encompasses derivatives, including the complementary sequence thereof.

By derivative is meant any single or multiple nucleotide deletions, additions or substitutions as well as mutants, fragments, portions or parts of the isolated nucleic acid molecule. All such deletions, additions, extensions, substitutions as well as mutants, fragments, portions, or parts are encompassed by the term "derivative". Therefore, the present invention also provides a nucleic acid construct comprising the abovementioned isolated polynucleotide. Useful derivatives include alterations to the 5'end portion of the polynucleotide sequence or the 3'end portion or a nucleotide sequence spanning the 5'and 3' portions. A derivative of the polynucleotide of the invention includes a polynucleotide hybridising selectively under stringent conditions with a polynucleotide with preferably between 90% and 100% sequence identity to the nucleotide sequence of SEQ ID NO: 3 or between 90% and 100% sequence identity to in particular the exon sequences of SEQ ID NO: 2 (residues 3963-4496, 4597-4661, 5976-6025, 6224-7321, 7533-7894, 8016-8841, 8933-9018 and 9145-9411 of SEQ ID NO: 2). A derivative of the nucleotide sequences of the invention also conveniently includes a nucleotide sequence having less than 100% sequence identity with an isolated polynucleotide which is selected from the group consisting of a polynucleotide with at least with preferably between 90% and 100% sequence identity to the nucleotide sequence of SEQ ID NO: 3 or in particular the exon sequences of SEQ ID NO: 2. but which is capable of hybridizing thereto under low stringency conditions at 42°C. A derivative of the nucleotide sequence set forth in SEQ ID NO: 2 or 3 preferably shows between 90 and 100% sequence identity to the nucleotide sequences set forth in SEQ ID NO: 3 or between 90 and 100% sequence identity to in particular the exon sequences of SEQ ID NO: 2. With respect to the nucleotide sequence of SEQ ID NO: 2 a derivative may have less than 90% sequence identity with the sequences as represented by residues 1-3962, 4497-4596, 4662-5975, 6026-6223, 7322-7532, 7895-8015, 8842-8932, 9019-9144, 9412-102282, because these sequences are not part of the coding sequence. In a derivative of SEQ ID NO:2 a sequence region, wherein the nucleic acid sequences at the positions which correspond, as determined by global sequence alignment, to the sequences as represented by residues 1-3962 (promoter region), 4497-4596, 4662-5975, 6026-6223, 7322-7532, 7895-8015, 8842-8932, 9019-9144 (introns) or 9412-102282 (terminator region) of SEQ ID NO: 2, may thus be longer, shorter, be less than 90 % homologous, or lack at all.

Sequence identity can be determined by alignment of two peptide or nucleotide sequences using global or local alignment algorithms. Sequences may then be referred to as 'substantially identical' when they (when optimally aligned by for example the programs GAP of BESTFIT using default parameters) share at least a certain minimal percentage of sequence identity such as 70% or 90 %. GAP uses the Needleman and Wunsch global alignment algorithm to align two sequences over their entire length, maximizing the number of matches and minimizing the number of gaps. Generally, the GAP default parameters are used, with a gap creation penalty = 50 for nucleotides/ 8 for protein and a gap extension penalty = 3 for nucleotides/ 2 for proteins. For nucleotides the default gap scoring matrix used in nwsgapdna (and for proteins the default scoring matrix is Blosum62 Henikoff & Henikoff, 1992). Sequence alignments and scores for percentage identity may be determined using computer programs such as NEEDLE from the EMBOSS package. Sequence identity may also be determined by searching against databases such as FASTA, BLAST etc. The skilled artisan will acknowledge that different parameters will yield slightly different results but that the overall percentage identity of two sequences will not be significantly altered when using different algorithms.

To be applied in the method of the invention, the isolated gene of the invention is cloned into a vector, such as a pMF1 vector (WO02097102), which is suitable for *Agrobacterium-*mediated transformation protocols. The invention thus also relates to a vector comprising the abovementioned gene. A selected vector preferably enables effective removal of undesired DNA sequences, like antibiotic resistance genes, from the obtained transgenic plants or plant tissues. In addition to the isolated polynucleotide of the invention a vector may further comprise the native promoter and terminator regions of the gene operably linked thereto.

The isolated gene of the invention, encoding the TIR NBS TRR protein (e.g. the *Vr2* gene) may be combined with a further gene encoding a protein that confers resistance against a pathogen in a plant, such as the HcrVf2 gene for resistance to *V*. *inaequalis.* Other genes that may be combined with the isolated gene of the invention may confer resistance against fire blight caused by the bacterium *Erwinia amylovora* or against mildew (*Podosphaera leucotricha*). The vector used to create a perennial plant with increased resistance against pathogens may thus comprise one or more further genes encoding proteins that confer resistance against scab or other diseases or pests.

Combining the isolated gene of the invention gene with other resistance genes, such as the *Vf*-gene (HcrVf2), enhances durability of resistance to (apple) scab. Alternatively, the isolated gene of the invention may be added to plants that have already other resistance genes.

Further, when the isolated gene is inserted into other genera such as pear (*Pyrus*) or peach (*Prunus*) it might provide also resistance to diseases (e.g. *Venturia pirina*) there.

### Example

A resistance gene appears to provide full resistance to apple scab, and there are no reports yet on breaking of this resistance. This gene originates from accession GMAL 2473, and was mapped on linkage group 2 (LG2) of apple (Patocchi et al. 2004). This gene is also named *Rvi15.* Galli et al. (2010a) developed additional markers around *Vr2*, and limited the genetic region in which the gene resides to 0.5 cM. Further, Galli et al. (2010b) isolated a bacterial artificial chromosome (BAC) spanning the mentioned genetic region of the resistance gene, using GMAL 2473 BAC libraries. This BAC clone was sequenced, allowing identification of the two markers flanking the resistance gene. Analysis of the 48.6-kb sequence between these markers revealed the presence of three putative genes characterized by a Toll and mammalian interleukin-1 receptor protein nucleotide-binding site leucine-rich repeat structure (TIR NBS LRR) that may be responsible for conferring resistance to apple scab. All three genes were found to be transcribed (Galli et al., 2010b).

### Cloning of the candidate genes.

The BAC clone from Galli et al. (2010b), its DNA sequence, and the prediction of the three putative candidate genes (Galli et al., 2010b) were used for designing primers for cloning. The native promoter region of approx. 3 kb and native terminator region of approx. 1 kb were included. The primers are shown in Table 1. The primers included restriction site for subsequent cutting and ligation steps.

The cloned DNA sequences of the three evaluated candidate genes of Vr2 are SEQ ID NO: 36 (*Vr2-A*)*,* SEQ ID NO: 37 (*Vr2-B*) and SEQ ID NO: 2 (*Vr2-C*). The coding sequence of *Vr2-C* was predicted using FGENESH at the Softberry-website to have the sequence as set forth in SEQ ID NO:3, encoding a protein with an amino acide sequence as set forth in SEQ ID NO: 1.

The amplification of the candidate genes was performed by DNA-Cloning Service (Hamburg, Germany), using template BAC-DNA and the primers mentioned in Table 1. Restriction sites were included in the primer sequences, as shown in Table 1. After the PCR-step the amplified DNA fragments were cloned into the pD1-*E*. *coli* cloning vector. To confirm that the DNA sequences did not contain any errors, the entire fragments were sequenced. Only error-free fragments were used for the further steps. The cloned fragments were excised from the cloning vector using the restriction enzymes mentioned in Table 1. The three fragments were individually ligated in the *AscI*-site of pMF1 and transferred to XL10gold *E. coli* cells for multiplication of the pMF1-Vr2 vector. The presence and orientation of the Vr2 candidate genes in the vectors was checked, using the primers mentioned in Table 2.

**Table 1. Primers, used to clone three candidate genes for Vr2.**

| **Primer name** | **Added restriction site** | **SEQ ID NO:** | **DNA sequence** |
|---|---|---|---|
| **Vr2-A-F** | BssH | **SEQ ID NO:38** | CCTTgcgcgccTTGCCGGAGATGAGAGAGA |
| **Vr2-A-R** | Mlu | **SEQ ID NO:39** | CCTTacgcgTCTAACCTTAACACTATGTGT |
| **Vr2-B-F** | BssH | **SEQ ID NO:40** | CCTTgcgcgccATTCCTCCAGCCCACCAA |
| **Vr2-B-R** | Mlu | **SEQ ID NO:41** | CCTTacgcgTGAATCTCCACCAGTAGAA |
| **Vr2-C-F** | BssH | **SEQ ID NO:42** | CCTTgcgcgccTGGTTGGTTCGGTTTAAGAC |
| **Vr2-C-R** | NheBss | **SEQ ID NO:43** | CCTTgctagcTgcgcgcGAATAAGTGGCAATGGTAGAAC |

**Table 2. Primers, used for checking the presence and orientation of the inserted Vr2-candidate genes in the pMF1 vector. The last two primers anneal to sites flanking the candidate genes.**

| **Primer name** | **SEQ ID NO:** | | **DNA sequence** |
|---|---|---|---|
| **Vr2-A-F9598** | **SEQ ID NO:44** | | TTCGATAATGGAGGCAAGTC |
| **Vr2-A-R239** | **SEQ ID NO:45** | | CAAGTTTTTGCGGTTTGGTT |
| **Vr2-B-F6746** | **SEQ ID NO:46** | | TAGGCGTTGGGCCATATTAG |
| **Vr2-B-R241** | **SEQ ID NO:47** | | GCAATTAGCTGTGCAAACCAT |
| **Vr2-C-F10427** | **SEQ ID NO:48** | | CATTTGCAAGACTCAGAAATGA |
| **Vr2-C-R241** | **SEQ ID NO:49** | | TCGAAATGTTTTCATTCCTTCC |
| **pBinPlusSeqRBside** | **SEQ ID NO:50** | | CTGCAAGGCGATTAAGTTGGGT |
| **pME1RecLBD** | **SEQ ID NO:51** | | CTTACCTACTGCTTCCAGAC |

### Transformation.

For transformation of apple, the pMF1-*Vr2* vectors (pMF1-*Vr2-A*, pMF1-*Vr2-B* and pMF1-*Vr2-C*) were transferred to the *Agrobacterium* strain Agl0 (Lazo et al. 1991). The transformation was performed as described by Joshi et al. (2011). The top four leaves of 4-week-old, in vitro propagated 'Gala' shoots were used as explants. The apple cultivar 'Gala' is very susceptible to apple scab (Joshi et al., 2011). For selection of transformants, kanamycin was used in the medium, as described by Joshi et al. (2011). For each construct, seven independent transformation events were selected and multiplied in vitro. The 'in vitro' grown plants were grafted directly onto rootstocks as described by Lane et al. (2003) and grown in the greenhouse, yielding one to four growing scions per event. The presence of T-DNA inserts was confirmed by isolating DNA from the leaves, and performing a PCR-analysis showing the presence of the CodA gene, which is part of the T-DNA insert. Also, non-transformed 'Gala' in vitro plantlets were micro-grafted, giving five growing scions.

### Scab resistance test.

When the scions had at least 10 leaves, scab disease tests were conducted in the temperature and humidity controlled greenhouse. A conidial suspension of a field population of V. *inaequalis* containing 5.10⁵ conidia/ml was prepared. The top two young, expanding leaves of the scions were sprayed with the conidial suspension till nearly run off. Inoculated plants were placed in a plastic tunnel at 100% relative humidity. The temperature in the greenhouse was set at 19°C during day and 16°C during night at a day-length of 16 h. After 48 hours, the plants were transferred outside the tunnel, but still kept in the same greenhouse compartment with the same temperature settings, and relative humidity of 85%.

Disease symptoms were assessed macroscopically 16 days post inoculation and classified in eight classes as described by Joshi et al. (2011), indicative for the amount of sporulation: class 0, 0% of sporulation; class 1, 1-2% sporulation; class 2, 2-5% sporulation; class 3, 5-10% sporulation; class 4, 10-25% sporulation; class 5, 25-50% sporulation; class 6, 50-75% sporulation; class 7, 75-100% sporulation. In addition to the quantitative sporulation scale, the types of resistance reactions were scored, using the scale of Chevalier et al. (1991).

### Results

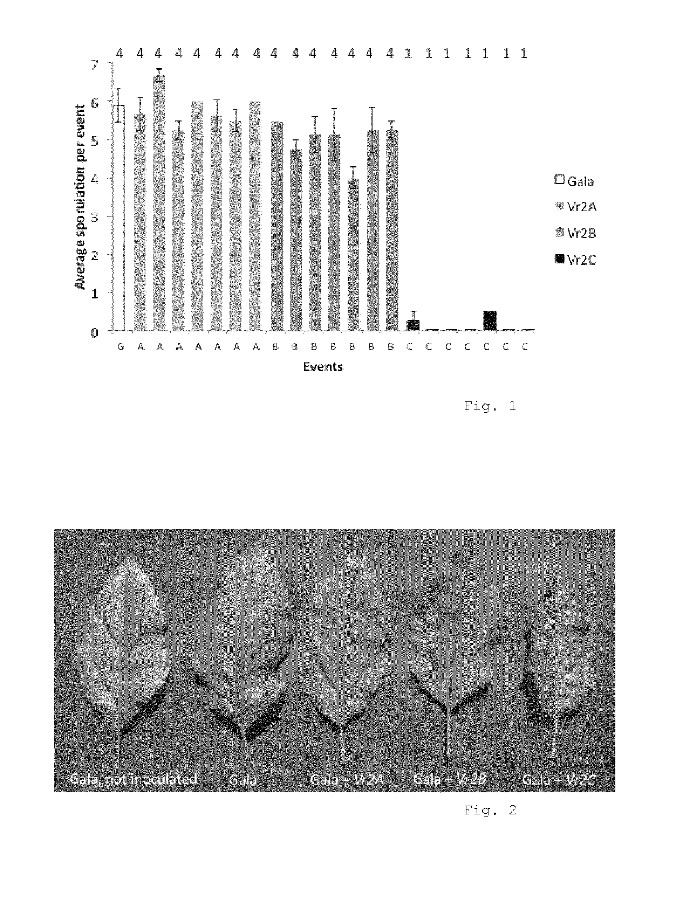

The result of the disease test is shown in Figure 1. Figure 1 shows the observed sporulation of the transformation events, containing candidate gene *Vr2-A, Vr2-B* or *Vr2-C.* Per construct, seven transformation events were grafted. One to four in vitro plantlets per event were micro-grafted, one per rootstock. For confirmation of the presence of the T-DNA insertion, leaves were collected from the scions of the transformed plants and non-transformed controls, for DNA extraction. Using this DNA, PCRs were performed using primers of the CodA gene, which is part of the T-DNA insert (WO02097102). All putatively transferred plants appeared to have the insert, apart from two escapes. The escapes were excluded from further analysis.

The sporulation data were averaged among the two upper leaves per scion, and the one to four scions per transformation event. Non-transformed 'Gala' was included as control. The sporulation was scored on a scale from 0 (no sporulation) till 7 (75-100 % of the leaf area covered with sporulation). The error bars display the standard deviations of the means of sporulation per plant. Above the bars, the symptom scores are displayed according to the scale of Chevalier et al. (1991). Symptom score 4 = susceptible; 1 = hypersensitive response resulting in pinpoint pits. The inoculated 'Gala' leaves without a *Vr2*-candidate gene, and the inoculated leaves containing *Vr2-A* or *Vr2-B* showed abundant sporulation (Figure 2). Figure 2 shows disease symptoms of leaves of the apple cultivar 'Gala' after inoculation with *Venturia inaequalis.* As control, a non-inoculated leaf of 'Gala' is shown. The inoculated leaf containing the *Vr2-C* candidate gene does not show any sporulation, but displays a hypersensitive response with pinpoint pits. *Vr2-A* and *Vr2-B* did not provide any visible resistance reaction. The *Vr2-C* events showed clearly pinpoint pits after inoculation, as shown in Figure 2. The pinpoint pits defence reactions provided by *Vr2-C* are also observed in the resistant donor plant GMAL2473 and resistant progeny from this parent (Patocchi et al., 2004; Galli et al., 2010a). Pinpoint pits are a result of a local collapse of upper epidermal cells, due to a hypersensitivity response (Chevalier et al., 1991). Figures 1 and 2 demonstrate that *Vr2-C* appears to be the functional *Vr2* resistance gene. The candidates *Vr2-A* and *Vr2-B* did not provide scab resistance.

In this example the cloning of the three candidate genes for *Vr2*, the insertion of the individual candidate genes into the susceptible cultivar 'Gala', using *Agrobacterium tumefaciens* mediated transformation, and testing of the resulting plants for resistance to apple scab is described. Only one of the candidate genes appeared to provide full resistance to apple scab, showing the same resistance reactions (hypersensitive response, leading to pinpoint pits) as observed in the *Vr2* donor plant GMAL 2473. The other candidate genes did not provide resistance to scab. The resistance reaction of *Vr2* is a hypersensitive response, giving a local collapse of upper epidermal cells, leading to pinpoint pits (Chevalier et al., 1991).

The identification and cloning of the *Vr2* resistance gene allows insertion of this gene into plants, such as susceptible apple cultivars, thus providing these plants resistance to apple scab. This approach prevents quality loss of the fruits due to linkage drag, and allows a strong reduction of fungicide input for control of apple scab. The identification of the functional gene also allows development of a perfectly linked genetic marker to *Vr2* resistance.

The isolated *Vr2*-gene as represented by sequences SEQ ID NO: 2 and SEQ ID NO: 3 thus allows enrichment of existing susceptible apple cultivars with resistance to *V. inaequalis.* This is not feasible by means of conventional breeding, as apple is self-incompatible, and therefore does not allow repeated backcrosses to restore the original genotype. Because of isolation of the *Vr2*-gene it is feasible now to insert this resistance gene into germplasm without linkage drag from the donor plant. Only the desired gene is inserted, without genetically linked undesired genes or alleles. This can save decennia of breeding time. For example, the introgression of the *HcrVf2* gene took more than 50 years, due to linkage drag. After isolation of this resistance gene, it took only a few years to insert it into a high quality cultivar, providing this cultivar resistance to apple scab (Joshi et al., 2011). A similar approach is now feasible for *Vr2*.

The isolated *Vr2*-gene allows now studying the resistance mechanism of this gene in greater detail and with more accuracy than was possible before, as now the gene can be inserted in the same genetic background as the susceptible comparator has. This is not feasible by means of crossings.

As the functional gene for the *Vr2* resistance is known, it is possible to develop genetic markers inside the gene rather than in the neighbourhood of the gene, using the known sequence of the *Vr2*-gene. Such markers are perfectly genetically linked to the resistance. Before the discovery of the functional *Vr2*-gene, the markers were in the vicinity of the gene, still allowing recombination between the gene and the marker.

The isolated *Vr2*-gene allows studying the function and mechanism of this gene in other species and other genera, after inserting this gene into these other organisms.

The isolated *Vr2*-gene allows production of pure Vr2 protein, using an expression vector.

### References

Anonymous. 1999. Variety List of Fruit Crops (in Dutch), Dutch Fruit Growers Organization.
Belfanti, E., Silfverberg-Dilworth, E., Tartarini, S., Patocchi, A., Barbieri, M., Zhu, J., Vinatzer, B.A., Gianfranceschi, L., Gessler, C. and Sansavini, S., 2004. The HcrVf2 gene from a wild apple confers scab resistance to a transgenic cultivated variety. Proc. Natl. Acad. Sci. USA 101:886-890.
Bus, V.G.M., Rikkerink, E.H.A., Caffier V., Durel C.E., and Plummer K.M. 2011. Revision of the nomenclature of the differential host-pathogen interactions of Venturia inaequalis and Malus. Annual review of phytopathology, 49, pp.391-413.
Calenge, F., Faure, A., Goerre, M., Gebhardt, C., van de Weg, W.E., Parisi, L. and Durel, C.E. 2004. Quantitative Trait Loci (QTL) analysis reveals both broad-spectrum and isolate-specific QTL for scab resistance in an apple progeny challenged with eight isolates of Venturia inaequalis. Phytopathology 94:370-379.
Chevalier, M., Lespinasse, Y. & Renaudin, S. (1991) A microscopic study of the different classes of symptoms coded by the Vf gene in apple for resistance to scab (Venturia inaequalis). Plant Pathol. 40, 249-256.
Crandall CS (1926) Apple breeding at the university of Illinois. Illinois Agric Exp Stn Bull 275:341-600.
Galli P., Broggini G.A.L., Kellerhals M, Gessler C., and Patocchi A. 2010a. High-resolution genetic map of the Rvi15 (Vr2) apple scab resistance locus. Molecular Breeding, pp.1-12.
Galli P., Patocchi A., Broggini G.A.L., and Gessler C. 2010b. The Rvi15 (Vr2) apple scab resistance locus contains three TIR-NBS-LRR genes. Molecular plant-microbe interactions : MPMI, 23(5), pp.608-617.
Gardiner, S.E., Bus, V.G.M., Rusholme, R.L., Chagné, D. and Rikkerink, E.H.A. 2006. Apple. p. 2-62. In: C. Kole (ed.), Genome Mapping and Molecular Breeding. Volume 4: Fruit and Nuts. Springer-Verlag: Berlin.
Gessler, C., Patocchi, A., Sansavini, S., Tartarini, S. and Gianfranceschi, L. 2006. Venturia inaequalis resistance in apple. Crit. Rev. Plant Sci. 25:473-503.
Gessler, C., Pertot, I., 2011. Vf scab resistance of Malus. Trees, 26(1), pp.95-108.
Henikoff & Henikoff, 1992, PNAS 89, 915-919
Hough, L.F., Shay, J.R. and Dayton, D.F. 1953. Apple scab resistance from Malus 202 floribunda Sieb. Proc. Amer. Soc. Hort. Sci. 62:341-347.
Joshi, S.G. et al., 2011. Functional analysis and expression profiling of HcrVf1 and HcrVf2 for development of scab resistant cisgenic and intragenic apples. Plant Molecular Biology, 75(6), pp.579-591.
Lane WD, Bhagwat B, Armstrong JD, Wahlgren S (2003) Apple micrografting protocol to establish transgenic clones on field ready rootstock. HortTechnology 13:641-646.
Lazo GR, Stein PA, Ludwig RA (1991) A DNA transformation competent Arabidopsis genomic library in Agrobacterium. Nat Biotechnol 9:963-967
Patocchi A, Bigler B, Koller B, Kellerhals M, Gessler C (2004) Vr2: a new apple scab resistance gene. Theor Appl Genet 109:1087-1092.
Parisi, L., Lespinasse, Y., Guillaumes, J. and Kruger, J. 1993. A new race of Venturia inaequalis virulent to apples with resistance due to the Vf gene. Phytopathology 83:533-537. Parisi, L., Laurens, F., Didelot, F., Evans, K., Fischer, C., Fouillet, V., Gennari, F., Kemp, H., Lateur, M., Patocchi, A., Schouten, H.J. and Tsipouridis, C. 2006. Geographical distribution of Venturia inaequalis strains virulent to the Vf gene in Europe. Bull. OILB/SROP 29:49-52.
Schouten, H.J., Krens, F.A. and Jacobsen, E. 2006a. Do cisgenic plants warrant less stringent oversight? Nature Biotechnol. 24:753.
Schouten, H.J., Krens, F.A. and Jacobsen, E. 2006b. Cisgenic plants are similar to traditionally bred plants. EMBO Rep. 7:750-753.
Schouten HJ, Soriano JM, Joshi SG, Kortstee AJ, Krens FA, Schaart JG, Van der Linden K, Allan AC, Hellens RP, Espley RV, and Jacobsen E 2009. Cisgenesis Is a Promising Approach for Fast, Acceptable and Safe Breeding of Pip Fruit. Acta Hort. 814, ISHS 2009, pp.199-204.
Trapman, M. 2006. Resistance management in Vf resistant organic apple orchards. Bull. OILB/SROP 29:253-257.

## Claims

1. A method for increasing resistance against a pathogen in a perennial plant, comprising incorporating into the genome of the perennial plant a gene encoding a protein having the sequence as set forth in SEQ ID NO: 1 or a sequence with at least 70% sequence identity thereto.

2. The method according to claim 1, wherein said gene is derived from a plant that is sexually compatible with said perennial plant.

3. The method according to claim 1 or 2, comprising incorporating at least one further pathogen resistance gene into the genome of said perennial plant.

4. The method according to claim 3, wherein the at least one further pathogen resistance gene is at least one gene selected from the group of *HcrVf2,* one or more genes conferring resistance against fire blight caused by the bacterium *Erwinia amylovora,* and one or more genes conferring resistance against mildew *(Podosphaera leucotricha).*

5. The method according to any of claims 1-4, wherein the perennial plant is selected from the group of apple (*Malus*), pear (*Pyrus*) or peach (*Prunus*).

6. The method according to claim 5, wherein the perennial plant is *Malus x domestica.*

7. The method according to any of claims 1-6, wherein said gene is derived from an apple scab resistant apple plant.

8. The method according to claim 7, wherein said apple scab resistant apple plant is GMAL 2473 (PI 589835, from the Geneva National Germplasm Repository).

9. The method according to any of claims 1-8, wherein said gene comprises a nucleotide sequence as set forth in SEQ ID NO: 3 or a sequence with at least 90% sequence identity to SEQ ID NO: 3.

10. The method according to any of claims 1-8, wherein said gene comprises a nucleotide sequence as set forth in positions 3963-9411 of SEQ ID NO: 2 or a sequence wherein the nucleic acid sequences at the positions which correspond, as determined by sequence alignment, to the nucleic acid sequences that are represented by residues 3963-4496, 4597-4661, 5976-6025, 6224-7321, 7533-7894, 8016-8841, 8933-9018 and 9145-9411 of SEQ ID NO: 2 are at least 90 % identical.

11. The method according to any of claims 1-10, wherein said pathogen is a *Venturia* species.

12. The method according to claim 11, wherein the pathogen is selected from the group comprising *Venturia inaequalis, Venturia pirina* and *Venturia carpophila.*

13. An isolated nucleic acid molecule derived from an apple scab resistant apple plant comprising a nucleotide sequence that encodes a protein having the sequence as set forth in SEQ ID NO: 1 or a sequence with at least 70% sequence identity thereto.

14. The isolated nucleic acid molecule according to claim 13, which comprises the sequence as set forth in SEQ ID NO: 3 or a sequence with at least 90% sequence identity to SEQ ID NO: 3; or a polynucleotide hybridising selectively under stringent conditions thereto, or a sequence complementary thereto.

15. The isolated nucleic acid molecule according to claim 13, which comprises the sequence as set forth in SEQ ID NO: 2 or a sequence wherein the nucleic acid sequences at the positions which correspond, as determined by sequence alignment, to the nucleic acid sequences that are represented by residues 3963-4496, 4597-4661, 5976-6025, 6224-7321, 7533-7894, 8016-8841, 8933-9018 and 9145-9411 of SEQ ID NO: 2 are at least 90 % identical; or a polynucleotide hybridising selectively under stringent conditions thereto, or a sequence complementary thereto.
